(19) Europäisches Patentamt / European Patent Office / Office européen des brevets

(11) **EP 4 285 818 A1**

(12) **EUROPEAN PATENT APPLICATION**

(43) Date of publication:
**06.12.2023 Bulletin 2023/49**

(21) Application number: **23176151.1**

(22) Date of filing: **30.05.2023**

(51) International Patent Classification (IPC):
**A61B 5/113** (2006.01)     **A61B 5/00** (2006.01)
**G16H 50/00** (2018.01)

(52) Cooperative Patent Classification (CPC):
**A61B 5/113; A61B 5/4809; A61B 5/4812;
A61B 5/681; A61B 5/7264; G16H 40/63;
G16H 50/20;** G16H 20/70

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **03.06.2022 US 202263365840 P
28.04.2023 US 202318309386**

(71) Applicant: **Apple Inc.
Cupertino CA 95014 (US)**

(72) Inventors:
• **MOLLAZADEH, Mohsen**
  **Cupertino, 95014 (US)**
• **KALIDAS, Vignesh**
  **Cupertino, 95014 (US)**
• **BAGHERZADEH, Nader E.**
  **Cupertino, 95014 (US)**

(74) Representative: **Barton, Russell Glen
Withers & Rogers LLP
2 London Bridge
London SE1 9RA (GB)**

(54) **SYSTEMS AND METHODS FOR SLEEP STATE TRACKING**

(57)    A wearable device including a motion-tracking sensor can be used for tracking sleep. The data from the motion-tracking sensor can be to estimate/classify the sleep state for multiple periods and/or to determine sleep intervals. In some examples, to improve performance, sleep state classification can be performed on data within a sleep tracking session. The start of the sleep tracking session can be defined by detecting a rest state and the end of the sleep tracking session can be defined by an activity state. In some examples, to improve performance, the classified sleep states for the multiple periods can be filtered and/or smoothed. In some examples, a signal quality check can be performed for the data from the motion-tracking sensor. In some examples, the classification of the sleep states and/or the display of the results of sleep tracking can be subject to passing one or more signal quality checks.

FIG. 1A

FIG. 1B

EP 4 285 818 A1

**Description**

**CROSS-REFERENCE TO RELATED APPLICATIONS**

This application claims the benefit of U.S. Provisional Application No. 63/365,840, filed June 3, 2022, and U.S. Patent Application No. 18/309,386, filed April 28, 2023, the contents of which are incorporated herein by reference in their entireties for all purposes.

**FIELD**

This relates generally to systems and methods for tracking sleep state, and more particularly, to tracking sleep state using a wearable device.

**BACKGROUND**

Sound sleep is considered vital for health. Abnormal sleep habits may lead to many health disorders. Some sleep disorders may adversely affect the physical and psychological functioning of human body. Accordingly, providing users with information about sleep state can be useful to improve sleep habits and health.

**SUMMARY**

This relates to systems and methods for tracking sleep using a wearable device. The wearable device can include one or more sensors including a motion (and/or orientation) tracking sensor (e.g., accelerometer, gyroscope, inertia-measurement unit (IMU), etc.) among other possible sensors. The data from the one or more sensors can be processed in the wearable device and/or by another device in communication with the one or more sensors of the wearable device to estimate/classify the sleep state for multiple periods and/or to determine sleep state intervals (e.g., during a sleep tracking session). In some examples, to improve performance, a sleep/wake classification can be performed on data from a sleep tracking session (e.g., classifying the sleep state as awake/wake or asleep/sleep). In some examples, to improve performance, a sleep/wake classification can be performed on data from a sleep tracking session to determine a more detailed sleep state (e.g., awake, rapid-eye-movement (REM) sleep, non-REM sleep stage one, non-REM sleep stage two, non-REM sleep stage three). The start of the sleep tracking session can be defined by detecting a rest state and the end of the sleep tracking session can be defined by an activity state. In some examples, to improve performance, the classified sleep states for the multiple periods can be filtered and/or smoothed. In some examples, a signal quality check can be performed for the data from the one or more sensors. In some examples, display of the results of sleep tracking can be subject to passing the signal quality check.

**BRIEF DESCRIPTION OF THE DRAWINGS**

FIGs. 1A-1B illustrate an example system that can be used to track sleep according to examples of the disclosure.

FIGs. 2A-2D illustrate example block diagrams and corresponding timing diagrams for sleep tracking according to examples of the disclosure.

FIG. 3 illustrates an example process for a rest/active classifier according to examples of the disclosure.

FIG. 4 illustrates an example process for a sleep/wake classifier according to examples of the disclosure.

FIG. 5 illustrates an example block diagram of feature extraction for sleep/wake classification according to examples of the disclosure.

FIG. 6 illustrates an example process for a quality check classifier according to examples of the disclosure.

FIGs. 7A-7B illustrate a block diagram for smoothing/filtering and a plot indicative of in-bed detection according to examples of the disclosure.

FIG. 8 illustrates an example process for a sleep state classifier according to examples of the disclosure.

**DETAILED DESCRIPTION**

In the following description of examples, reference is made to the accompanying drawings which form a part hereof, and in which it is shown by way of illustration specific examples that can be practiced. It is to be understood that other examples can be used and structural changes can be made without departing from the scope of the disclosed examples.

This relates to systems and methods for tracking sleep using a wearable device. The wearable device can include one or more sensors including a motion (and/or orientation) tracking sensor (e.g., accelerometer, gyroscope, inertia-measurement unit (IMU), etc.) among other possible sensors. The data from the one or more sensors can be processed in the wearable device and/or by another device in communication with the one or more sensors of the wearable device to estimate/classify the sleep state for multiple periods and/or to determine sleep state intervals (e.g., during a sleep tracking session). In some examples, to improve performance, a sleep/wake classification can be performed on data from a sleep tracking session (e.g., classifying the sleep state as awake/wake or asleep/sleep). In some examples, to improve performance, a sleep/wake classification can be performed on data from a sleep tracking session to determine a more detailed sleep state (e.g., awake, rapid-eye-movement (REM) sleep, non-REM sleep stage one, non-REM sleep stage two, non-REM sleep stage three). The more detailed sleep state classification is often referred to herein as sleep state classification (performed by a sleep state classifier), but may be understood as a more detailed example of a sleep/wake classification. The start of the sleep tracking session can be defined by detecting a rest state and the end of the sleep tracking session can be defined by an activity state. In some examples, to improve performance, the classified sleep states for the multiple periods can be filtered and/or smoothed. In some examples, a signal quality check can be performed for the data from the one or more sensors. In some examples, display of the results of sleep tracking can be subject to passing the signal quality check.

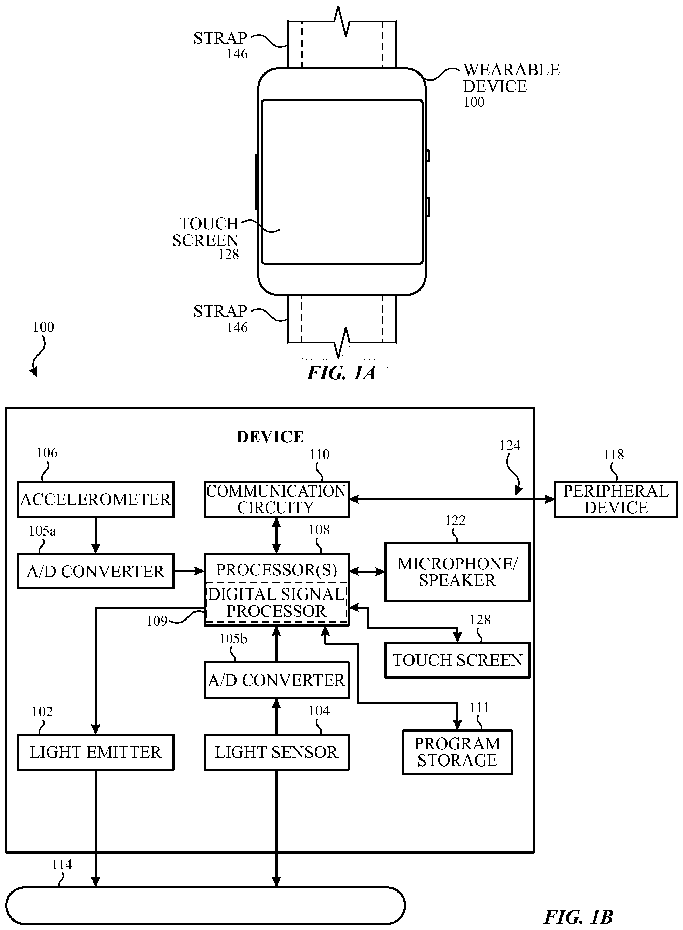

FIGs. 1A-1B illustrate an example system that can be used to track sleep according to examples of the disclosure. The system can include one or more sensors and processing circuitry to estimate/classify sleep state for multiple periods using the data from the one or more sensors. In some examples, the system can be implemented in a wearable device (e.g., wearable device 100). In some examples, the system can be implemented in more than one device (e.g., wearable device 100 and a second device in communication with wearable device 100).

FIG. 1A illustrates an example wearable device 100 that can be attached to a user using a strap 146 or other fastener. Wearable device 100 can include one or more sensors used to estimate/classify the sleep state for multiple periods and/or to determine sleep intervals, and optionally can include a touch screen 128 to display the results of sleep tracking as described herein.

FIG. 1B illustrates an example block diagram of the architecture of wearable device 100 used to track sleep according to examples of the disclosure. As illustrated in FIG. 1B, the wearable device 100 can include a one or more sensors. For example, the wearable device 100 can optionally include an optical sensor including one or more light emitter(s) 102 (e.g., one or more light emitting diodes (LEDs)) and one or more light sensor(s) 104 (e.g., one or more photodetectors/photodiodes). The one or more light emitters can produce light in ranges corresponding to infrared (IR), green, amber, blue and/or red light, among other possibilities. The optical sensor can be used to emit light into a user's skin 114 and detect reflections of the light back from the skin. The optical sensor measurements by the light sensor(s) can be converted to digital signals (e.g., a time domain photoplethysmography (PPG) signal) for processing via an analog-to-digital converter (ADC) 105b. The optical sensor and processing of optical signals by the one or more processors 108 can be used, in some examples, for various functions including estimating physiological characteristics (e.g., heart rate, arterial oxygen saturation, etc.) or detecting contact with the user (e.g., on-wrist/off-wrist detection).

The one or more sensors can include a motion-tracking and/or orientation-tracking sensor such as an accelerometer, a gyroscope, an inertia-measurement unit (IMU), etc. For example, the wearable device 100 can include accelerometer 106 that can be a multi-channel accelerometer (e.g., a 3-axis accelerometer). As described in more detail herein, the motion-tracking and/or orientation-tracking sensor can be used to extract motion and respiration features used to estimate sleep state. Measurements by accelerometer 106 can be converted to digital signals for processing via an ADC 105a.

The wearable device 100 can also optionally include other sensors including, but not limited to, a photothermal sensor, a magnetometer, a barometer, a compass, a proximity sensor, a camera, an ambient light sensor, a thermometer, a global position system sensor, and various system sensors which can sense remaining battery life, power consumption, processor speed, CPU load, and the like. Although various sensors are described, it is understood that fewer, more, or different sensors may be used.

The data acquired from the one or more sensors (e.g., motion data, optical data, etc.) can be stored in memory in wearable device 100. For example, wearable device 100 can include a data buffer (or other volatile or non-volatile memory or storage) to store temporarily (or permanently) the data from the sensors for processing by processing circuitry. In some examples, volatile or non-volatile memory or storage can be used to store partially processed data (e.g., filtered data, down-sampled data, extracted features, etc.) for subsequent processing or fully processed data for storage of sleep tracking results and/or display or reporting sleep tracking results to the user.

The wearable device 100 can also include processing circuitry. The processing circuitry can include one or more processors 108. One or more of the processors can include a digital signal processor (DSP) 109, a microprocessor, a central processing unit (CPU), a programmable logic device (PLD), a field programmable gate array (FPGA), and/or the like. In some examples, the wearable device 100 can include a host processor and a low-power processor. The low-power processor may be a continuously powered processor and the host processor may be powered up or powered down depending on a mode of operation. For example, a low-power processor can sample accelerometer 106 while a user is sleeping (e.g., when the host processor may be powered off), whereas the host processor can perform some or all of the sleep/wake classification or sleep state classification at the conclusion of the sleep tracking session (e.g., when the host processor may be powered on). The various processing and classifiers described in more detail herein can be implemented entirely in the low-power processor, entirely in the host processor, or implemented partially in both the low-power processor and the host processor.

In some examples, some of the sensing and/or some of the processing can be performed by a peripheral device 118 in communication with the wearable device. The peripheral device 118 can be a smart phone, media player, tablet computer, desktop computer, laptop computer, data server, cloud storage service, or any other portable or non-portable electronic computing device (including a second wearable device). The peripheral device may include one or more sensors (e.g., a motion sensor, etc.) to provide input for one of the classifiers described herein and processing circuitry to perform some of the processing functions described herein. Wearable device 100 can also include communication circuitry 110 to communicatively couple to the peripheral device 118 via wired or wireless communication links 124. For example, the communication circuitry 110 can include circuitry for one or more wireless communication protocols including cellular, Bluetooth, Wi-Fi, etc.

In some examples, wearable device 100 can include a touch screen 128 to display the sleep tracking results (e.g., displaying sleep intervals and/or total sleep time for a sleep tracking session, optionally with the detail of sleep time for different sleep state intervals) and/or to receive input from a user. In some examples, touch screen 128 may be replaced by a non-touch sensitive display or the touch and/or display functionality can be implemented in another device. In some examples, wearable device 100 can include a microphone/speaker 122 for audio input/output functionality, haptic circuitry to provide haptic feedback to the user, and/or other sensors and input/output devices. Wearable device 100 can also include an energy storage device (e.g., a battery) to provide a power supply for the components of wearable device 100.

The one or more processors 108 (also referred to herein as processing circuitry) can be connected to program storage 111 and can be configured to (programmed to) to execute instructions stored in program storage 111 (e.g., a non-transitory computer-readable storage medium). The processing circuitry, for example, can provide control and data signals to generate a display image on touch screen 128, such as a display image of a user interface (UI), optionally including results for a sleep tracking session. The processing circuitry can also receive touch input from touch screen 128. The touch input can be used by computer programs stored in program storage 111 to perform actions that can include, but are not limited to, moving an object such as a cursor or pointer, scrolling or panning, adjusting control settings, opening a file or document, viewing a menu, making a selection, executing instructions, operating a peripheral device connected to the host device, answering a telephone call, placing a telephone call, terminating a telephone call, changing the volume or audio settings, storing information related to telephone communications such as addresses, frequently dialed numbers, received calls, missed calls, logging onto a computer or a computer network, permitting authorized individuals access to restricted areas of the computer or computer network, loading a user profile associated with a user's preferred arrangement of the computer desktop, permitting access to web content, launching a particular program, encrypting or decoding a message, and/or the like. The processing circuitry can also perform additional functions that may not be related to touch processing and display. In some examples, processing circuitry can perform some of the signal processing functions (e.g., classification) described herein.

Note that one or more of the functions described herein, including sleep tracking (e.g., sleep/wake classification, sleep state classification), can be performed by firmware stored in memory or instructions stored in program storage 111 and executed by the processing circuitry. The firmware can also be stored and/or transported within any non-transitory computer-readable storage medium for use by or in connection with an instruction execution system, apparatus, or device, such as a computer-based system, processor-containing system, or other system that can fetch the instructions from the instruction execution system, apparatus, or device and execute the instructions. In the context of this document, a "non-transitory computer-readable storage medium" can be any medium (excluding signals) that can contain or store the program for use by or in connection with the instruction execution system, apparatus, or device. The computer-readable storage medium can include, but is not limited to, an electronic, magnetic, optical, electromagnetic, infrared, or semiconductor system, apparatus or device, a portable computer diskette (magnetic), a random access memory (RAM) (magnetic), a read-only memory (ROM) (magnetic), an erasable programmable read-only memory (EPROM) (magnetic), or flash memory such as compact flash cards, secured digital cards, universal serial bus (USB) memory devices, memory sticks, and the like.

The firmware can also be propagated within any transport medium for use by or in connection with an instruction execution system, apparatus, or device, such as a computer-based system, processor-containing system, or other system that can fetch the instructions from the instruction execution system, apparatus, or device and execute the instructions. In the context of this document, a "transport medium" can be any medium that can communicate, propagate, or transport the program for use by or in connection with the instruction execution system, apparatus, or device. The transport medium can include, but is not limited to, an electronic, magnetic, optical, electromagnetic, or infrared wired or wireless propagation medium.

It should be apparent that the architecture shown in FIG. 1B is only one example architecture, and that the wearable device could have more or fewer components than shown, or a different configuration of components. The various components shown in FIG. 1B can be implemented in hardware, software, firmware, or any combination thereof, including one or more signal processing and/or application specific integrated circuits. Additionally, the components illustrated in FIG. 1B can be included within a single device or can be distributed between multiple devices.

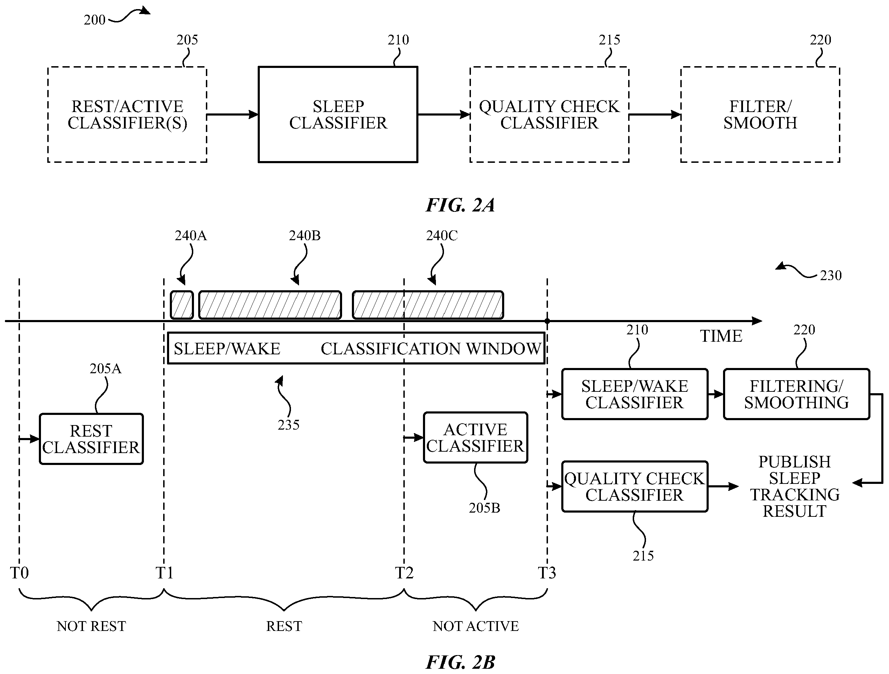

FIGs. 2A-2D illustrate example block diagrams and corresponding timing diagrams for sleep tracking according to examples of the disclosure. FIGs. 2A-2B illustrate an example block diagram and corresponding timing diagram for sleep tracking (e.g., sleep/wake classification) according to examples of the disclosure. FIG. 2A illustrates an example block diagram 200 of processing circuitry for sleep tracking of according to examples of the disclosure. The processing circuitry can include a digital signal processor (e.g., corresponding to DSP 109 in FIG. 1B) and/or one or more additional processors (e.g., corresponding to processor(s) 108). In some examples, the processing circuitry can include a programmable logic device (PLD), field programmable gate array (FPGA), or other logic device. The processing circuitry can include a rest/active classifier 205, a sleep/wake classifier 210, a quality check classifier 215, and a smoothing/filtering post-processor 220. The classifications and/or filtering/smoothing can be implemented in hardware, software, firmware, or any combination thereof.

Rest/active classifier 205 can be optionally included as part of sleep tracking to bound the data to be stored and/or processed for sleep/wake classification (potentially reducing the storage and/or processing requirements and power consumption for the sleep tracking system). In particular, the rest/active classifier 205 can be used to define a start time for a sleep tracking session (e.g., corresponding to an estimation/classification that a user is resting) and/or an end time for a sleep tracking session (e.g., corresponding to an estimation that the user is active and not resting or sleeping). The bounding of the sleep tracking session assumes that a user is unlikely to be sleeping while active/not-resting. In some examples, the rest/active classifier 205 can be implemented as one or more classifiers (e.g., a separate rest classifier and a separate active classifier). In some examples, the same classifier can be used but different thresholds can be used for rest classification before the start of a sleep tracking session than used for active classification during the sleep tracking session.

Quality check classifier 215 can be optionally included for sleep tracking to estimate/classify the quality of the sensor data (e.g., using one or more features extracted during the sleep/wake classification). The quality of the sensor data can be indicative of the wearable device being on-wrist during the sleep tracking session and can establish a confidence in the sleep/wake classification. Smoothing and filtering post-processor 220 can optionally be included to smooth/filter the sleep/wake classification.

FIG. 2B illustrates an example timing diagram 230 illustrating features and operation of the processing circuitry for sleep tracking according to examples of the disclosure. At time T0, the rest classifier (e.g., the rest/active classifier using the "rest" thresholding parameters) can be triggered and can begin processing input data in accordance with process 300 to detect whether a user is resting or not (e.g., in a rest state or active state). In some examples, the rest classification can begin in response to satisfaction of one or more first triggering criteria. The one or more first triggering criteria can include a first trigger criterion that is met at a pre-defined time or in response to a user input. For example, the rest classifier can be triggered at a user-designated "bedtime" (or a default bedtime if the sleep tracking feature is enabled for the system without the user designating a bedtime) or a predefined time (e.g., 120 minutes, 90 minutes, 60 min, 45, minutes, 30 minutes, etc.) before the user-designated bedtime (or default bedtime). In some examples, the rest classifier can be triggered by a user request to perform a sleep tracking session (or an indication that the user is currently in-bed or plans to go to bed soon). In some examples, in addition to the first trigger criterion, the rest classifier can process input only after an indication that the wearable device is worn by the user (or the absence of an indication that the wearable device is not off-wrist). For example, the one or more first triggering criteria can further include a second criterion that is satisfied when detecting that the wearable device is on-wrist (e.g., using the optical sensor or other sensor). The one or more first triggering criteria can further include a third criterion that is satisfied when detecting that the wearable device is not charging (e.g., via an inductive charger). Although three example criteria are described, it is understood that fewer, more, or different criteria can be used in some examples. In some examples, the rest classifier can process data until the rest classifier indicates that the user is in a rest state (at T1). T1 can define the start of a session. In some examples, the rest classifier can process data until a timeout occurs, at which time the sleep tracking can be terminated.

In some examples, at time T2, an active classifier (e.g., the rest/active classifier using the "active" thresholding parameters) can be triggered and can begin processing input data in accordance with process 300 to detect whether the user is active or not (e.g., in an active state or a rest state). In some examples, the active classifier can begin in response to satisfaction of one or more second triggering criteria. The one or more second triggering criteria can include a first trigger criterion that is met at a pre-defined time or in response to a user input. For example, active classifier can be triggered at a user-designated "wake-up time" (or a default wake-up time) or a predefined time (e.g., 120 minutes, 90 minutes, 60 min, 45, minutes, 30 minutes, etc.) before a user-designated "wake-up time" (or default wake-up time). In some examples, the active classifier can process data until the active classifier indicates that the user is in an active state. In some examples, after the active state is indicated by the active classifier, the user can be presented with a notification and the user input in response (e.g., tapping a button on the touch screen of the wearable device) can confirm the active state. In some examples, the active state (and its confirmation via user input if implemented) can define the end of the session. As illustrated in FIG. 2B, T3 can define the end of a session.

In some examples, the session can be terminated in other ways. In some examples, the session can be terminated upon dismissal of an alarm, detecting that the wearable device is off-wrist (e.g., using the optical sensor or other sensor), detecting that the wearable device is charging, a session timeout (e.g., a threshold time after T1 or after a threshold time after a user-designated wake-up time), a user input to end a session, or detecting an active state classification by the active classifier after a user-designated wake-up time, among other possibilities.

As shown in FIG. 2B, the session can be defined by the start time T1 and the end time T3. The data collected in the period in between T1 and T3 can be included in the sleep/wake classification window 235. Although FIG. 2B defines the sleep/wake classification window 235 between T1 and T3, in some examples, the sleep/wake classification window 235 can begin earlier. In some examples, the sleep/wake classification window can begin at T0. In some examples, the sleep/wake classification window can begin some threshold period of time before T1. For example, the threshold period of time can be the same as the first period used for thresholding at 335 in process 300, described below.

The data in the sleep/wake classification window 235 can be processed by the sleep/wake classifier 210 as described in more detail with respect to process 400 and block diagram 500. In some examples, the sleep/wake classification by sleep/wake classifier 210 can begin in response to the end of the session (or a threshold period of time after the session or in response to a user request). In some examples, the sleep/wake classification by sleep/wake classifier 210 can begin only after the confidence in the session is satisfied as determined by the quality check classifier 215. In some examples, the sleep/wake classification by sleep/wake classifier 210 can begin (e.g., upon the end of the session), but can be aborted if ongoing, if the confidence in the session is not satisfied as determined by the quality check classifier. In some examples, the sleep/wake classification estimating a user's sleep state can be stored in memory and/or displayed to the user. For example, sleep/wake classification estimating a user's sleep state can be displayed or stored as a sequence of sleep intervals (e.g., consecutive periods of time classified as the sleep state) represented by blocks 240A-240C as shown on the timeline in FIG. 2B.

Although as described above the rest classifier runs for a period (e.g., from T0 to T1) and the active classifier runs for a period (e.g., starting at T2, and until T3), in some examples, the rest/active classifier can run for longer durations. For example, the rest/active classifier can run continuously (e.g., 24 hours a day, optionally only while the wearable device is on-wrist and/or not charging) or the rest/active classifier can run continuously between the user-defined bedtime and wake-up (or a threshold time before and/or after the user-define bedtime/wake-up), and multiple sleep/rest classification windows can be identified (rather than the one window illustrated in FIG. 2B). The samples from each identified sleep/rest classification window can be processed and tried to identify sleep intervals, as described herein. In some examples, rather than operating continuously, the operation of the rest/active classifier can be periodic, intermittent or in response to one or more triggers.

In some examples, the sleep/wake classification estimating a user's sleep state can be displayed and/or stored only when confidence in the session is satisfied as indicated by quality check classifier 215. The quality check by quality check classifier 215 can begin in response to the end of the session. In some examples, the quality check classifier can estimate whether the motion data collected by the wearable device corresponds to the wearable device remaining on-wrist during the session (e.g., between the indication of on-wrist by an optical sensor). Using motion data can save power and reduce light while a user is sleeping as compared with using the optical sensor for on-wrist detection during the sleep tracking session.

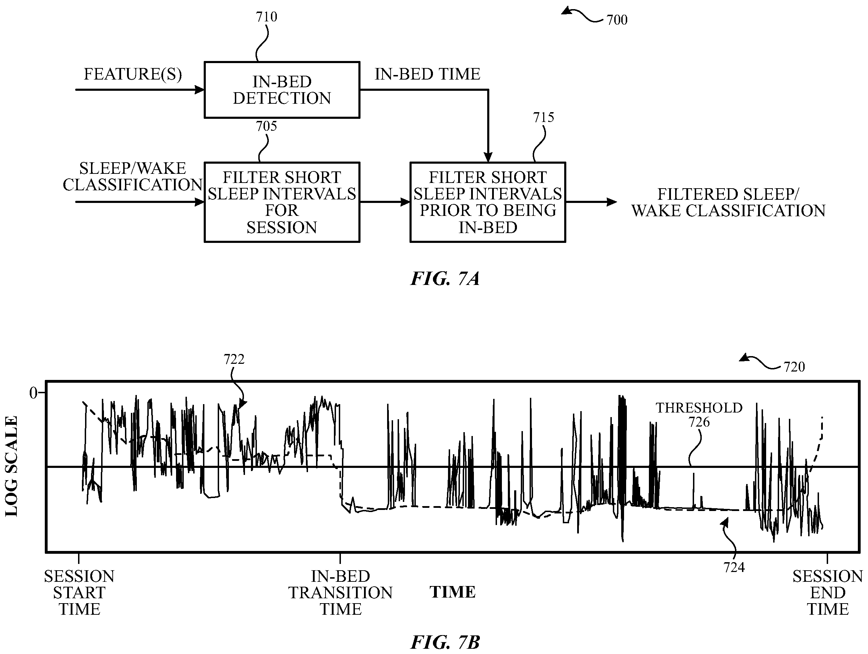

In some examples, the sleep/wake classification estimating a user's sleep state can be smoothed or filtered by smoothing/filtering post-processor 220 to remove indications of very short durations of sleep that may be incorrect due to the presence of quiet wakefulness (e.g., awake periods with respiration and motion features indicative of sleep, but prior to onset of sleep). The smoothing and filtering by smoothing/filtering post-processor 220 is described in more detail with respect to FIGs. 7A-7B. In some examples, the smoothing/filtering can be performed on the output of sleep/wake classifier 210 only after the quality check is satisfied (e.g., to avoid filtering/smoothing when the sleep/wake classifications will not be displayed and/or stored).

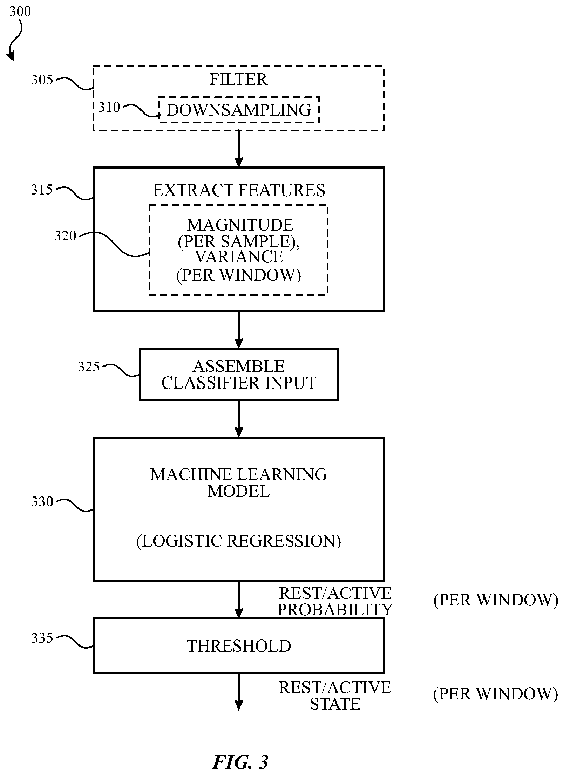

FIG. 3 illustrates an example process for a rest/active classifier according to examples of the disclosure. Process 300 can be performed by processing circuitry including processor(s) 108 and/or DSP 109. Process 300 can be performed in real-time (e.g., as sufficient data for processing is received) once the rest/active classification is triggered (e.g., in accordance with satisfying one or more first/second triggering criteria). At 305, the rest/active classifier can optionally filter the data input into the classifier. The data can include motion data from a three-axis accelerometer (or other suitable motion and/or orientation sensor). In some examples, the filtering can be a low-pass filter to filter out high frequency noise (e.g., outside of the frequency of expected user motion). In some examples, the motion data can also be down-sampled at 310. For example, the accelerometer may capture motion data at a first sampling rate (e.g., 60 Hz, 100 Hz, 125Hz, 250Hz, etc.) and the motion data can be down-sampled (e.g., multi-stage polyphase filter) to a lower rate (e.g., 4 Hz, 8 Hz, 10 Hz, 30 Hz, 50 Hz, etc.). Down-sampling the motion data can reduce the number of samples and thereby reduce the processing complexity. In some examples, the motion data can be processed without down-sampling and/or without filtering.

At 315, the rest/active classifier can extract one or more features from the motion data. In some examples, the one or more features can be extracted for samples in each "rest/active classifier window" or simply "window" in the context of rest/active classification (e.g., distinct from an "epoch" which can be a longer duration window for sleep/wake classification or sleep state classification). For example, the motion data be divided into N non-overlapping windows that include M samples of acceleration in each dimension (X, Y, Z) of a three-channel accelerometer. In some examples, the window can be between 1-30 seconds in duration. In some examples, the window can be between 1-10 seconds in duration. In some examples, the window can be between 2-5 seconds.

In some examples, the one or more features can include a magnitude feature for each sample in the window and a variance feature for the samples in the window (320). The magnitude of each of the M samples in a window can be computed using equation (1):

$$magnitude = \sqrt{X^2 + Y^2 + Z^2} \qquad (1)$$

where X, Y and Z represent the x-axis accelerometer measurement for a sample, y-axis accelerometer measurement for a sample, and z-axis accelerometer measurement for a sample, respectively. The variance of the M magnitude values for the window can be computed using equation (2):

$$\sigma^2 = \frac{\sum_{i=1}^{M}(mag_i - \overline{mag})^2}{M} \qquad (2)$$

where $\sigma^2$ represents the variance for the window, M represents the number of samples in the window, $mag_i$ represents the magnitude of the $i^{th}$ sample, and $\overline{mag}$ represents the mean magnitude for the window.

At 325, the input for the classifier can be assembled. The rest/active classifier input can be assembled from features for N windows and thus the input can correspond to a longer duration period than the window used for extraction of the magnitude and variance features described above (e.g., corresponding to periods of 30 seconds, 60 seconds, 90 seconds, 120 seconds, etc.). In some examples, the input can include N*(M+1) features. In some examples, the input can be compressed to reduce the number of features. For example, the features from multiple windows can be reduced by sum-pooling the features for k consecutive windows to reduce the input to $\frac{N}{k} * (M + 1)$ features. In some examples, k can be between 2-10. In some examples, k can be between 3-8. A buffer can be used to store data (raw acceleration data and/or extracted magnitude and variance features) corresponding to the longer duration period such that sufficient data can be available as input to the rest/active classifier.

At 330, the classifier input can be processed with a machine-learning (ML) model, such as a logistic regression. It is understood that logistic regression is just one example of an ML model and other models can be used such as gradient-boosted trees, random forests, neural networks, support vector machines, etc. The output of the ML model can be a confidence value representing the probability (between 0 and 1) that the user is in a resting state. In some examples, the ML model can output a confidence value for each period of time corresponding to the duration of the window (e.g., using a sliding window on the data buffer). For example, a first input of N windows (e.g., windows 1-100) can be used to calculate a first confidence value, a second input of N windows (e.g., windows 2-101) can be used to calculate a second confidence value, and so on. Thus, the output of the ML model can be represented as an array of confidence values (per window).

At 335, a threshold can be applied to the output of the ML model to detect a rest or an active state, with different parameters used for rest classification than for active classification. For example, a rest state (e.g., for the rest classification beginning at T0 in FIG. 2B) can be detected when the rest confidence value is greater than a first threshold confidence value for a first threshold number of windows in a given first period. For example, the rest state can be detected when the rest state confidence is greater than the first threshold confidence value (e.g., 85%, 90%, 95%, etc.) for most or all of (e.g., for 95%, 100%, etc. of) a first period (e.g., of a duration of 3 minutes, 5, minutes, 10 minutes, etc.). An active state (e.g., for the active classification beginning at T2 in FIG. 2B) can be detected when the rest confidence value is less than a second threshold confidence value for a second threshold number of windows in a given second period. For example, the active state can be detected when the rest state confidence is less than the second threshold confidence value (e.g., 70%, 75%, 80%, etc.) for (e.g., for 10%, 15%, etc. of) a second period (e.g., of a duration of 15 minutes, 20, minutes, 30 minutes, etc.). In some examples, the first threshold confidence value and the second threshold confidence value can be the same. In some examples, the first threshold confidence value and the second threshold confidence value can be different such that it may require a relatively higher confidence of rest to enter the rest state (from the non-resting/active state) and a relatively lower confidence of rest to enter the active state (from the non-active/rest state). In some examples, detecting the rest state can require the first threshold number of windows in the first period be consecutive (e.g., a threshold number of consecutive minutes with a rest state confidence above the threshold), whereas detecting the active state may not require the second threshold number of windows in the second period be consecutive (e.g., a threshold number of consecutive or non-consecutive minutes of activity within a longer period).

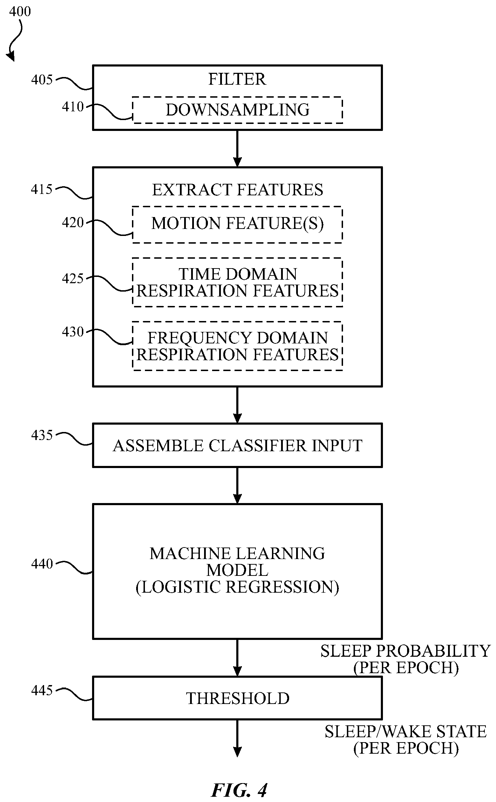

FIG. 4 illustrates an example process for a sleep/wake classifier according to examples of the disclosure. Process 400 can be performed by processing circuitry including processor(s) 108 and/or DSP 109. In some examples, process 400 can be performed partially in real-time (e.g., as sufficient data for processing is received), partially in a cadence during the session, and/or partially at the end of the session. In some examples, process 400 can be performed entirely at the end of the session.

At 405, the sleep/wake classifier can optionally filter the data input into the classifier. The data can include motion data from a three-axis accelerometer (or other suitable motion and/or orientation sensor). In some examples, the filtering can be a low-pass filter to filter out high frequency noise (e.g., outside of the frequency of expected user motion/respiration). In some examples, the motion data can also be down-sampled at 410. For example, the accelerometer may capture motion data at a first sampling rate (e.g., 60 Hz, 100 Hz, 125Hz, 250Hz, etc.) and the motion data can be down-sampled (e.g., multi-stage polyphase filter) to a lower rate (e.g., 4 Hz, 8 Hz, 10 Hz, 30 Hz, 50 Hz, etc.). In some examples, down-sampling and low-pass filtering can be performed in real-time or in a cadence during the session to reduce the amount of data to be processed and/or stored. In some examples, the motion data can be processed without down-sampling and/or without low-pass filtering.

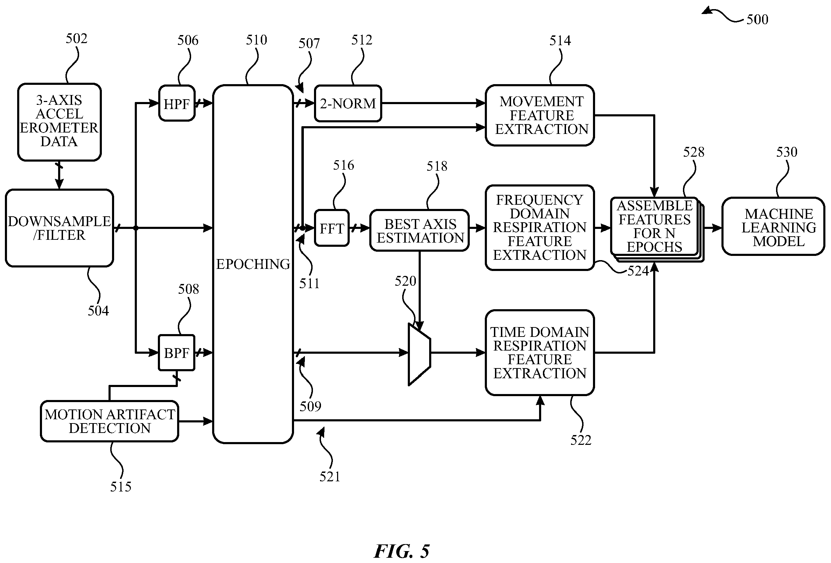

At 415, the sleep/wake classifier can extract multiple features from the motion data. In some examples, the one or more features can include one or more motion features (420), one or more time-domain respiration features (425), and one or more frequency-domain respiration features (430). The multiple features can be computed for each epoch of motion data. The epoch can represent a window of motion data samples for sleep/wake classification (e.g., a sleep/wake classifier window) with a duration greater than the duration of the window used for rest/active classification (e.g., the rest/active classifier window). In some examples, the epoch can represent a window with a duration the same as the duration of the window used for rest/active classification. In some examples, the epoch can be between 10-120 seconds in duration. In some examples, the epoch can be between 30-90 seconds in duration. In some examples, the epoch can be between 45-60 seconds. In some examples, the feature extraction can be performed on epochs that define overlapping periods. For example, adjacent epochs can overlap by 5-60 seconds. In some examples, the overlap can be between 20-30 seconds. Feature extraction is described in more detail herein with respect to FIG. 5.

At 435, the input for the sleep/wake classifier can be assembled. The sleep/wake classifier input can be assembled from features for N epochs and can correspond to a longer duration period (e.g., corresponding to 5 minutes, 10 minutes, etc.). In some examples, the input can include N*M features, where M features are extracted for each of N epochs. In some examples, the N epochs include an epoch of interest (e.g., the one for which the output classification applies) and N-1 epochs before and/or after the epoch of interest. In some examples, (N-1)/2 epochs before the epoch of interest are used as well as (N-1)/2 epochs after the epoch of interest. In some examples, the N-1 epochs may not be distributed evenly on both sides of the epoch of interest (e.g., 75% before and 25% after the epoch of interest). In some examples, the N-1 epochs before the epoch of interest are used. In some examples, the input can be compressed to reduce the number of features. For example, the features from multiple epochs can be reduced by sum-pooling the features for k consecutive epochs to reduce the input to $\frac{N}{k} * M$ features. A buffer can be used to store data (raw and/or filtered/down-sampled acceleration data and/or extracted features) corresponding to the longer duration period such that sufficient data can be available as input to the sleep/wake classifier.

At 440, the classifier input can be processed with a ML model, such as a logistic regression. It is understood that logistic regression is just one example of an ML model and other models can be used such as gradient-boosted trees, random forests, neural networks, support vector machines, etc. The output of the ML model can be a confidence value representing the probability (between 0 and 1) that the user is in a sleep state. In some examples, the ML model can output a confidence value for each period of time corresponding to the duration of the epoch (e.g., using a sliding window on the data buffer). For example, a first input of N epochs (e.g., epochs 1-20) can be used to calculate a first confidence value, a second input of N epochs (e.g., epochs 2-21) can be used to calculate a second confidence value, and so on. Thus, the output of the ML model can be represented as an array of confidence values (per epoch).

At 445, a threshold can be applied to the output of the ML model to detect a sleep or a wake state. For example, a sleep state can be detected when the sleep confidence value is greater than a threshold confidence value and the wake state can be detected when the sleep confidence value is less than the threshold. In some examples, the threshold can be set based on the machine learning model and training data to maximize Cohen's kappa. The output of thresholding can be an array of sleep/wake state classifications (per epoch). The array of sleep/wake state classifications can be displayed (optionally with some post-processing and in accordance with a quality check) as sleep intervals (e.g., a sequence of sleep and awake periods) as described herein.

FIG. 5 illustrates an example block diagram of feature extraction for sleep/wake classification (or sleep state classification) according to examples of the disclosure. Block diagram 500 illustrates input motion data 502 from a three-axis accelerometer (e.g., a three-channel motion sensor) that can be taken from a raw data buffer and/or from the output of ADC 105a. The input motion data can be down-sampled and/or low-pass filtered in a down-sampling and/or filtering block 504 (e.g., implemented in a hardware or software). The extraction of features from the motion data can be performed from different streams of the motion data. The one or more motion features can be extracted by the motion feature extraction block 514 from a 3-axis stream of the motion data further filtered by a high-pass filter 506 and/or from a 3-axis stream of the motion data without the further high-pass filtering. The one or more time-domain respiration features can be extracted by the time-domain respiration feature extraction block 522 from one selected axis of a 3-axis stream of the motion data further filtered using a band-pass filter 508. The one or more frequency domain respiration features can be extracted by the frequency-domain respiration feature extraction block 524 from one selected axis of a 3-axis stream of the motion data without the further high-pass filtering. The selection of the one axis of the 3-axis stream can be performed using the 3-axis stream of the motion data without the further high-pass filtering. In some examples, high-pass filter 506 can filter out some or all of the respiration band (e.g., filter out data below a threshold frequency, such as 0.5Hz), and band-pass filter 508 can filter out some or all data outside the respiration band (e.g., passing data between in a range of frequencies, such as between 0.1 Hz and 0.6 Hz).

The motion data can be divided into epochs for feature extraction by an epoching block 510 (e.g., implemented in hardware or software). In some examples, the epoching can be achieved using a sliding window of the duration of an epoch (e.g., accessing motion data from a data buffer corresponding to the epoch duration). The epoching can be performed on multiple streams of the accelerometer data including the 3-axis high-pass filtered accelerometer stream (output by high-pass filter 506), the 3-axis band-pass filtered accelerometer stream (output by band-pass filter 508), and the 3-axis accelerometer stream without high-pass or band-pass filtering (output by down-sampling and/or filtering block 504).

The one or more motion features extracted by the motion feature extraction block 514 can include a "maximum variance" motion feature. The maximum variance can be computed from among the epoched 3-axis accelerometer stream 511 (without high-pass filtering or band-pass filtering). The variance of the magnitude for samples in the epoch be computed for each channel of the epoched 3-axis accelerometer stream 511 in a similar manner as described above in equation (2), but for the single-axis magnitude of each sample in the epoch. The maximum variance among the three variance values for the 3-channels of epoched 3-axis accelerometer stream 511 (e.g., a first variance value for a first channel, a second variance value for the second channel and a third variance for the third channel) can represent the maximum variance feature. Additionally or alternatively, in some examples, a natural logarithm of the maximum variance feature may be used as a motion feature.

The one or more motion features extracted by the motion feature extraction block 514 can include a "mean variance" motion feature. The magnitude (2-norm) of motion for each sample in the epoched high-pass filtered 3-axis accelerometer stream 507 can be computed in a 2-Norm magnitude block 512 (e.g., in a similar manner as described in equation (1) as applied to the epoched high-pass filtered 3-axis accelerometer stream 507). In some examples, the magnitude can be computed for the high-pass filtered 3-axis accelerometer stream prior to epoching (e.g., on a sample-by-sample basis). The variance of the magnitude for each of the samples in the epoch be computed. The mean variance feature can be computed as the mean of the computed variances across all the samples in the epoch. The mean variance feature can correlate with a wake state. Although described as a mean variance motion feature, additionally or alternatively, the one or more features extracted by the motion feature extraction block 514 can include the median variance or the mode variance (e.g., taking a median or a mode of the variances across all the samples in the epoch).

The one or more motion features extracted by the motion feature extraction block 514 can include a "motion count" motion feature. The motion count feature can be a determination of the number of motion samples in the epoch with magnitude of motion above a threshold. The magnitude (2-norm) of motion for each sample in the epoched high-pass filtered 3-axis accelerometer stream 507 can be computed in a 2-Norm magnitude block 512 (e.g., in a similar manner as described in equation (1) as applied to the epoched high-pass filtered 3-axis accelerometer stream 507). In some examples, the magnitude can be computed for the high-pass filtered 3-axis accelerometer stream prior to epoching (e.g., on a sample-by-sample basis). The motion count feature can be determined by counting the number of samples or the fraction/percentage of samples in the epoch whose 2-norm magnitude of motion is above a threshold. The motion count feature can indicate an amount of motion above some noise threshold for the epoch.

The one or more motion features extracted by the motion feature extraction block 514 can include a "motion integration" motion feature. The motion integration feature can sum the magnitudes for the sample in the epoch by integrating the magnitudes as scaled by a dx term (e.g., $\int magnitude \bullet dx$), where dx can be the sampling period (inverse of the sampling rate after down-sampling). The magnitude (2-norm) of motion for each sample in the epoched high-pass filtered 3-axis accelerometer stream 507 can be computed in the 2-Norm magnitude block 512 as described above. The motion integration feature can indicate the overall magnitude of motion for the epoch. The motion integration feature can be useful for identifying slower, sustained movements in the epoch, whereas the motion count feature can be useful for identifying faster movements (e.g., higher frequency movements/transients).

The one or more motion features extracted by the motion feature extraction block 514 can include a "motion integration mean" motion feature. The motion integration mean feature can be a mean of the "motion integration" feature described above. The motion integration mean feature can indicate the average of the overall variability in the magnitude of motion for the epoch. The motion integration mean feature can be useful for potentially identifying short-term, high-motion segments, which may correspond to short wake bouts. Although described as a motion integration mean feature, additionally or alternatively, the one or more features extracted by the motion feature extraction block 514 can include the motion integration median or the motion integration maximum.

The above motion features are examples of one or more motion features that could be extracted by the motion feature extraction block 514. It is understood that addition, fewer, and/or different motion features can be extracted for use in sleep/wake classification. In some examples, the sleep/wake classification can use the "maximum variance" feature, the "motion count" feature, and the "motion integration" feature. In some examples, the sleep state classification described with reference to process 800 can further use the "mean variance" feature and the "motion integration mean" feature.

The one or more frequency-domain respiration features extracted by the frequency-domain respiration feature extraction block 524 can include one or more measures of the variability in a motion-sensor-derived respiration signal. In some examples, the one or more features can be computed from one-axis of the epoched 3-axis accelerometer stream 511 (without high-pass filtering or band-pass filtering). The one-axis of the epoched 3-axis accelerometer stream 511 can be selected for each epoch by the best axis estimation block 518 as the axis with the best respiration signal (e.g., based on a signal-to-noise ratio (SNR)). A frequency domain representation can be computed for each axis of the epoched 3-axis accelerometer stream 511 in order to determine a best respiration signal. For example, a Fourier transform (e.g., using fast Fourier transform (FFT) block 516) can be computed for each axis and/or a power spectral density (PSD) can be computed for each axis. In some examples, the mean can optionally be subtracted from the epoched 3-axis accelerometer stream 511 before computing the frequency domain representation (e.g., de-trending). An SNR can be computed for each axis of the 3-axis accelerometer stream 511 based on the frequency representation. The "signal" of the SNR can be estimated by identifying a maximum peak in the frequency representation and computing spectral power (absolute value squared of the FFT) within a frequency-domain window around the maximum peak (e.g., within a range of a fundamental frequency). In some examples, a folded spectrum can be computed by summing the power over one or more harmonics of the frequency-domain window (e.g., optionally including some of the side-lobe bins around the fundamental frequency), and the spectral power can be computed based on the largest peak in the folded spectrum (e.g., the dominant frequency across multiple harmonics) and summing the power over the multiple harmonics including the side-lobe bins of the dominant frequency. In some examples, the "noise" of the SNR can be estimated by computing the spectral power outside the frequency-domain window around the maximum peak. The SNR can be computed from the ratio of the above defined signal and noise. The axis with the best respiration signal can be selected for an epoch based on the axis with the maximum SNR among the three axes for the epoch.

It should be understood that the above description of determining the SNR is an example, and the SNR can be computed in other ways and/or the axis with the best respiration signal can be determined in other ways. For example, the SNR can be computed in some examples as the log of the ratio of the "signal" described above to the total power of the spectrum (without computing the noise as described above). In some examples, rather than computing the best axis, the respiration signal can be extracted using singular spectrum analysis (SSA), principal component analysis (PCA), or rotation angles (RA). However, the above SNR approach can reduce processing complexity relative to SSA, PCA and RA, while providing the desired performance for sleep/wake classification.

In some examples, the frequency-domain respiration features can include one or more "spectral power" respiration features for the selected best axis for one or more frequency ranges. The power spectral density (PSD) can be computed from the epoched 3-axis accelerometer stream 511 (e.g., using FFT block 516), optionally after de-trending. The spectral power feature can be a relative spectral density computed by the expression: $\frac{band\ power}{total\ power}$, where the band power can be computed by integrating the PSD within the frequency limits of the band and the total power can be computed by integrating the total PSD. In some examples, the extraction of frequency-domain respiration features can include computing a first relative spectral power in the frequency range (e.g., 0.01-0.04 Hz), a second relative spectral power in the frequency range (e.g., 0.04-0.1 Hz), a third relative spectral power in the frequency range (e.g., 0.1-0.4 Hz), and a fourth relative spectral power in the frequency range (e.g., 0.4-0.9 Hz). The relative spectral density features can be useful for sleep/wake classification because heart rate and/or respiration rate can have different modulations of power in these different frequency bands for a sleep state as compared with an awake state.

In some examples, the frequency-domain respiration features can include a "spectral entropy" respiration feature. The spectral entropy feature can be calculated from the selected best axis (optionally after de-trending). For example, the PSD can be calculated from an FFT, and the spectral entropy can be calculated from the PSD. For example, the spectral entropy can be calculated by normalizing the PSD (e.g., to sum to 1), treating the normalized PSD as a probability density function (PDF), and computing the Shannon Entropy. The spectral entropy can be useful for sleep/wake classification because a more regular breathing pattern associated with sleep can include a sharper PSD and therefore a lower spectral entropy.

In some examples, the frequency-domain respiration features can include a "respiration rate" respiration feature. The respiration rate feature can be calculated from the selected best axis (optionally after de-trending). In some examples, the frequency domain representation of the best axis can be computed using an FFT, and a frequency with the highest peak in the spectral output of the FFT can be identified as the respiration rate. Calculating the respiration rate in frequency domain can provide for a more robust measurement (e.g., less susceptible to noise) compared with the time domain. In some examples, the respiration rate can be converted to a number-of-breaths per period of time (e.g., per minute). The respiration rate can be useful to identify sleep state due to an understanding of how respiration rate changes in different stages of sleep.

The above frequency-domain respiration features are examples of one or more frequency-domain respiration features that could be extracted by the frequency-domain respiration feature extraction block 524. It is understood that addition, fewer, and/or different frequency-domain respiration features can be extracted for use in sleep/wake classification. In some examples, the sleep/wake classification can use the "spectral power" feature and the "spectral entropy" feature. In some examples, the sleep state classification described with reference to process 800 can further use the "respiration rate" feature.

Time-domain respiration feature extraction block 522 can extract one or more time-domain respiration features. Extracting time-domain respiration features can be based on identifying peak and valley indices in the epoched band-pass filtered 3-axis accelerometer stream 509 and time intervals between peaks and valleys. The peaks and valleys can be associated with inhales and exhales (with the amplitude associated with breath intensity), and the time intervals between the peaks and valleys can be associated with breath times and durations. In some examples, these quantities can be extracted for the epoch, and the most stable quantities among these can be used for subsequent time-domain feature extraction, as described in more detail below.

In some examples, the one or more time-domain respiration features can be computed from one-axis of the epoched, band-pass filtered 3-axis accelerometer stream 509, where the one axis is selected is accordance with the operation of best axis estimation block 518. This selection is illustrated in FIG. 5 by multiplexer 520 receiving a control signal from best axis estimation block 518 to select one axis of the epoched 3-axis accelerometer stream 509 to use for time-domain respiration feature extraction.

Because the time-domain respiration features are extracted from motion data (e.g., one selected axis of the epoched 3-axis accelerometer stream 509), the respiration signal can be susceptible to motion artifacts (e.g., motion unrelated to respiration). In some examples, the presence of a motion artifact can be estimated by motion artifact detection block 515 using the 3-axis output of the band-pass filter 508. Motion artifact detection block 515 can compute a maximum absolute variance across the 3-axis band-pass filtered accelerometer stream in a similar manner to maximum variance motion feature described above. However, rather than computing one maximum variance for an epoch as described for the maximum variance motion feature, the maximum absolute variance computed by motion artifact detection block 515 can using a sliding window smaller than an epoch. In some examples, the sliding window can be between 1-10 seconds in duration. In some examples, the sliding window can be between 2-5 seconds in duration. In some examples, the sliding window can have the same duration as the rest/active classifier window. After computing the maximum absolute variance using the sliding window, the maximum absolute variances for multiple windows can be thresholded. For example, the motion artifact detection block 515 can output an array of binary values (a binary array) with a binary output value indicative of a motion artifact for the window when the maximum absolute variance is above a threshold (e.g., "1") and a binary output indicative of a no motion artifact for the window when the maximum absolute variance is below the threshold (e.g., "0"). The output of the motion artifact detection block 515 can be sampled at the same rate as the output of down-sampling and/or filtering block 504 (though the maximum absolute variances were determined on a per-window basis with each window including multiple samples). In some examples, to mitigate the effect of filter transients, the samples indicative of a motion artifact in the binary array can be "padded" such that a threshold number (e.g., 2, 3, 5, 8, 10, etc.) of samples on either side of a sample indicative of a motion artifact can also be marked as indicative of a motion artifact (even though the maximum absolute variance of the sample may be below the threshold). The output of the motion artifact detection block 515 can be epoched and passed as a motion artifact signal flag array 521 to time domain respiration feature extraction block 522 for time-domain respiration feature extraction. The motion artifact signal flag array 521 can mark portions of the one selected axis of the epoched 3-axis accelerometer stream 509 that can be excluded from the time-domain respiration feature extraction. For example, motion artifact signal flag array 521 can serve as a per-sample mask to suppress artifacts during respiratory peak/valley detection and/or to clean up breath locations and/or intervals. Although motion artifact detection is shown in FIG. 5 as occurring before epoching, it should be understood that in some examples, the generation of the motion artifact signal flag array 521 can be performed after epoching.

As described herein, time domain respiration features can be based on peaks and valleys detected in the selected axis of the epoched 3-axis accelerometer stream 509. The samples in an epoch that are not masked by the motion artifact signal flag array 521 (which are filtered out) can be processed to identify peak and valley locations with amplitudes (absolute value) above a threshold. In some examples, the threshold can be determined on a per-epoch basis by computing the standard deviation of the selected axis of the epoched 3-axis accelerometer stream 509 and multiplying the standard deviation by a scaling parameter. In some examples, the scaling parameter can be 1. In some examples, the scaling parameter can be greater than one or less than 1.

After computing the peaks and valleys (filtered for motion artifacts), inter-breath intervals (IBIs) can be computed by taking time differences between adjacent peak timestamps (inter-peak intervals) and/or the time difference between adjacent valley timestamps (inter-valley intervals). The IBIs can be indexed for storage using the interval start timestamps (e.g., peak start timestamps or valley start timestamps).

The identified peaks and valleys as well as the inter-peak intervals and inter-valley intervals can be filtered to remove portions from samples of the epoch that are contaminated by motion artifacts (e.g., using motion artifact signal flag array 521). For example, a peak that overlaps at least partially with samples contaminated by motion artifacts, a valley that overlaps at least partially with samples contaminated by motion artifacts, or an IBI that overlaps with motion artifacts can filtered out (e.g., to ensure that both the start point and end point of each breathing interval is free from motion artifacts). For example, a peak or valley may be detected at or near samples contaminated with motion artifacts can be masked out and/or breath intervals contaminated with motion artifacts can be masked out.

For the feature extraction, either the peaks (and inter-peak intervals) or the valleys (and inter-valley intervals) can be selected based on which show less variability. In some examples, the variability can be determined based on a standard deviation or a median absolute derivation of the IBIs within each epoch. For example, peaks (and inter-peak intervals) can be used if the variability for inter-peak intervals is lower than the variability for inter-valley intervals for the epoch, or the valleys (and inter-valley intervals) can be used if the variability for inter-valley intervals is lower than the variability for inter-peak intervals.

The one or more time-domain respiration features can include a "number of breaths" respiration feature indicating a number-of-breaths detected for the epoch, that can be determined by counting the number of peaks or valleys after the peak/valley and IBI detection and motion artifact filtering described above. The one or more time-domain respiration features can include a "respiratory amplitude variability" respiration feature for the epoch. The respiratory amplitude variability feature can be computed by computing the standard deviation of the amplitude of the peaks (or valleys) and normalizing the standard deviation of the amplitude of the peaks (or valleys) by the mean of the amplitude of the peaks (or valleys). In some examples, the one or more time-domain respiration features can include a "respiratory amplitude median" respiration feature for the epoch. The respiratory amplitude median feature can be computed by computing the median of the amplitude of the peaks (or valleys). In some examples, the one or more time-domain respiration features can include a respiratory amplitude mean (e.g., mean of the amplitude of the peaks (or valleys)) and/or a respiratory amplitude mode (e.g., mode of the amplitude of the peaks (or valleys)).

The one or more time-domain respiration features can include one or more respiratory rate variability (breath-to-breath variability) features for the epoch. A first respiratory rate variability feature can be a "mean-normalized median absolute deviation" respiration feature. This first respiratory rate variability feature can be computed by taking the difference between the instantaneous IBI and the median IBI for the epoch, and then normalizing by the mean IBI for the epoch. A second respiratory rate variability feature can be a "mean-normalized range" respiration feature. This second respiratory rate variability feature can be computed by taking the difference between the maximum and minimum IBI values for the epoch, and then normalizing by the mean IBI for the epoch. A third respiratory rate variability feature can be a "standard deviation" respiration feature. This third respiratory rate variability feature can be computed by taking the standard deviation of the IBI values for the epoch. A fourth respiratory rate variability feature can be a "root mean squared of successive differences" respiration feature. This fourth respiratory rate variability feature can be computed by taking the root-mean-squared deviations between successive peaks (or valleys) for the epoch.

Due to the motion artifact filtering or due to no breaths being detected in the epoch, in some examples, there may be insufficient data to compute one or more of the time-domain respiration features (e.g., except for the number of breaths feature, which is zero in such a case). For such epochs, the features can be assigned with predetermined values that correspond to a relatively high likelihood of a wake state (e.g., based on the empirical data). In some examples, predetermined values can be a percentile (e.g., 75th percentile, 85th percentile, 95th percentile) for each feature in the empirical data for a person who is awake.

The above time-domain respiration features are examples of one or more time-domain respiration features that could be extracted by the time-domain respiration feature extraction block 522. It is understood that addition, fewer, and/or different time-domain respiration features can be extracted for use in sleep/wake classification. In some examples, the sleep/wake classification can use the "number of breaths" feature, the "respiratory amplitude variability" feature, the "mean-normalized median absolute deviation" feature, the "mean-normalized range" feature, the "standard deviation" feature. In some examples, the sleep state classification described with reference to process 800 can further use the "root mean square of successive differences" feature and the "respiration amplitude median" feature.

The extracted features from multiple epochs can be assembled 528 (e.g., as described in process 400 at 435). In some examples, assembling can include sum-pooling. In some examples, assembling can include storing the extracted features (e.g., in a data buffer) for input into the machine learning model (e.g., logistic regression classifier). Logistic regression by sleep/wake classifier 530 can process the input to classify the input from multiple epochs (e.g., as described in process 400 at 440).

Referring back to FIGs 2A-2B, a quality check classifier 215 can optionally be included to establish a confidence in the sleep/wake classification. In particular, the quality check classifier 215 can evaluate one or more extracted features to provide a confidence in the motion data (e.g., indicative that the wearable device was worn by the user during the sleep/wake classification window 235). In some examples, the quality check classifier can use a subset of the multiple features used for sleep/wake classification. In some examples, the quality check classifier can use one or more extracted motion features, one or more time-domain respiration features, and one or more frequency-domain respiration features.

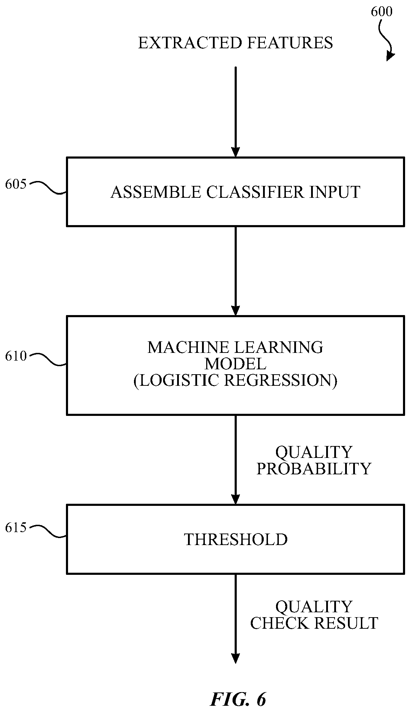

FIG. 6 illustrates an example process for a quality check classifier according to examples of the disclosure. Process 600 can be performed by processing circuitry including processor(s) 108 and/or DSP 109. In some examples, process 600 can be performed at the end of the session before, after or in parallel with the sleep/wake classification of process 400. In some examples, the subset of features can include the motion integration feature and the maximum variance motion feature. In some examples, the subset of features can include the spectral entropy feature and one (or more) of the relative spectral power features. In some examples, the subset of features can include a number-of-breaths per epoch feature. Using a subset of extracted features may be useful for reducing the size of the classifier input and therefore the complexity of the quality check classifier. Additionally, using extracted features from sleep/wake classification can avoid the need to extract additional features. In some examples, the same features extracted for sleep/wake classification may be used for the quality check classifier.

At 605, the input for the quality check classifier can be assembled. The quality check classifier input can be assembled from a subset of extracted features for the multiple epochs of the sleep/wake classification window. In some examples, the subset of extracted features for all epochs of the sleep/wake classification window can be used for quality check classification. In some examples, the input can be compressed to reduce the number of features. For example, the features from multiple epochs can be reduced by sum-pooling the features for k consecutive epochs to reduce the input by a factor of-.

At 610, the classifier input can be processed with a ML model, such as a logistic regression. It is understood that logistic regression is just one example of an ML model and other models can be used such as gradient-boosted trees, random forests, neural networks, support vector machines, etc. The output of the ML model can be a confidence value representing the probability (between 0 and 1) that the motion data is of a quality that it can pass the quality check (thereby expressing confidence in the sleep/wake classification based on the motion data). This quality check confidence value can correspond to the probability that the wearable device remained on-wrist (e.g., was not removed and resting on a table or other surface during the sleep/wake classification window) during the sleep/wake classification window.

At 615, a threshold can be applied to the output of the ML model to detect a quality check result or state. For example, the quality check can be passed (passed state) when the quality confidence value is greater than a threshold confidence value, and the quality check can be failed (failed state) when the quality confidence value is less than the threshold. As described herein, failing the quality check can result in forgoing published the sleep tracking results to the user (and/or discarding the sleep tracking results), whereas passing the quality check can result in storing and/or publishing the sleep tracking results.

Referring again back to FIGs 2A-2B, in some examples, a smoothing and filtering post-processor 220 can optionally be included to smooth/filter the sleep/wake classification output. FIGs. 7A-7B illustrate a block diagram 700 for smoothing/filtering and a plot 720 indicative of in-bed detection according to examples of the disclosure. In some examples, a first filter block 705 can filter the output of the sleep/wake classifier to remove very short sleep intervals (e.g., less than a threshold time such as 15 seconds, 30 seconds, 45 seconds, etc.) at any point in the session (e.g., across the entire sleep/wake classification window). These very short sleep intervals may be false positives (high-frequency transients) and/or may represent sleep intervals that are not sufficiently long for meaningful sleep/health benefits. These very short sleep intervals may also be difficult to present to the user because these representations of less meaningful sleep information to user clutters the presentation of the more meaningful longer duration sleep intervals in the sleep tracking result. Filtering out the very short sleep intervals can include replacing the indication of a sleep state of the very short sleep intervals with an indication of the awake state.

In some examples, the smoothing/filtering can include removing short sleep intervals in a portion of the session that may be indicative of rest rather than sleep. The portion of the session may refer to a time between the indication of a rest state (e.g., at T1 in FIG. 2B) and a detection that a user is "in bed" at some point during the sleep session (e.g., after T1 but before T2 in FIG. 2B). For example, one or more features extracted above for sleep/wake classification can be used for in-bed detection by in-bed detection block 710. In-bed detection block 710 can estimate a time (e.g., an epoch) in the session in which the user transitions from being "out of bed" to being "in-bed." The states of "out of bed" and "in-bed" may be defined as a function of movement rather than actually detecting whether the user is actually in a bed or not. In some examples, the one or more features can include the maximum variance motion feature extracted by the motion feature extraction block 514. The maximum variance motion feature can be filtered and the transition to the "in-bed" state can be detected when the filtered feature drops below a threshold. In some examples, the threshold can be a user-specific threshold.

In some examples, a log10 scale of the maximum variance motion feature can be used for in-bed detection (e.g., by taking the log base 10 of the maximum variance motion feature across the epochs of the session). For example, FIG. 7B illustrates a plot 720 with an example of a signal 722 corresponding to the log10-scaled maximum variance motion feature between the session start time and the session end time. In some examples, this log10-scaled maximum variance motion feature can be used to determine a user-specific threshold. The user-specific threshold can be set as the maximum between a default threshold (e.g., applicable to most users as defined by empirical data) and a threshold percentile (e.g., 55th percentile, 60th percentile, 65th percentile, etc.) of the log10-scaled maximum variance motion feature. In some examples, the default threshold can be used without determining or using a user-specific threshold.

The log10-scaled maximum variance motion feature can be filtered with a sliding window median-filter. The sliding window for in-bed detection can correspond to the duration of multiple epochs (e.g., 20, 50, 80, 100, 125, etc.) For the filtering, the session can be padded with zeroes on both ends (indicative of high levels of activity in log base 10 scale). FIG. 7B illustrates signal 724 corresponding to the median-filtered log10-scaled maximum variance motion feature (shown in dashed-line).

The epoch in which the median-filtered, log10-scaled maximum variance motion feature falls below the threshold can be detected as the in-bed transition epoch. For example, FIG. 7B illustrates threshold 726, and the epoch at the in-bed transition time indicated where the median-filtered, log10-scaled maximum variance motion feature crosses threshold 726.

In some examples, a second filter block 715 shown in FIG. 7A can filter the output of the sleep/wake classifier to remove short sleep intervals that correspond to quiet wakefulness that might be interpreted as false-positive sleep intervals. The second filter block 715 can filter out the short sleep intervals during the period between the start of the session and the in-bed transition epoch indicated by the in-bed detection block 710. In some examples, second filter block 715 can identify the short sleep intervals by identifying intervals of sleep that satisfy one or more interval criteria. The one or more interval criteria can include a first criterion that the sleep interval is less that a threshold duration (e.g., less than 5 minutes, less than 10 minutes, less than 20 minutes, etc.). The one or more interval criteria can include a second criterion that the sleep density within a period of time is less than a threshold sleep density (10%, 20%, 30%, etc.). The sleep density can be computed by examining a sleep interval and a period of time around the sleep interval to determine a percentage of the epochs in the period of time that indicate the sleep state. Sleep intervals that meet the one or more criteria can be removed (e.g., the sleep/wake classification for the interval can be changed from a sleep state to a wake state.

After filtering the sleep/wake classification using the first filter block 705 and/or the second filter block 715, the sleep/wake classification can be represented as sleep intervals and stored in memory and/or presented to the user (e.g., displayed on the touch screen). In some examples, the sleep intervals can be defined by the start time and end time of a group of sleep-classified epochs. In some examples, the sleep intervals can be displayed as a sequence or timeline. In some examples, the total sleep time from the sleep intervals can be summed and presented to the user as a total sleep time for the session in addition to, or instead of, the sleep intervals.

Although rest/active classifier(s), the sleep/wake classifier, and the signal quality classifier described herein use only motion data from a motion sensor (e.g., a 3-axis accelerometer). It is understood that, in some examples, these classifiers include additional sensor inputs to improve some or all of these classifiers to improve the overall sleep/wake classification for the system. However, using only motion data can provide a low-power (and/or low-light) classification without the use of additional sensor. In some examples, respiration features can be extracted from other sensors (e.g., using an optical sensor to extract respiration features (such as heart rate and heart rate variability features) from a photoplethysmography (PPG) signal or electrocardiogram (ECG) signal). In some examples, a sensor strip (e.g., including one or more sensors such as piezoelectric sensors and/or proximity sensor(s)) on or in a bed can be used to detect respiration signals and/or motion signals for extraction of features (to improve performance and/or confidence of the rest/active classification, sleep/wake classification, and/or quality check classification) and/or to detect in-bed conditions (e.g., for in-bed detection). In some examples, user inputs or states of the wearable device or another device (e.g., wearable device 100 and peripheral device 118) can be used as inputs as well. For example, user input to unlock/lock and/or to interact with the touchscreen or other input devices of the wearable device or a mobile phone or tablet computing device in communication with the wearable device can be used as indicators that a user is not in a sleep state (e.g., in a wake state and/or active state). This information can be used to correct incorrect classifications (e.g., false-positive sleep state classification) and/or can be used to forgo processing data to extract features and/or classify epoch when the contextual cues indicate an awake state.

As described herein, the processing of motion data for feature extraction can be done in real-time or in a cadence during operation. In some examples, the rest/active classifier can operate in real-time or in a cadence (e.g., during operation from T0 to T1 and/or from T2 to T3 illustrated in FIG. 2 B). In some examples, the sleep/wake classifier, the quality check classifier and the filtering/smoothing post-processing can be performed at the end of the session. In some examples, the feature extraction for sleep/wake classifier and/or the quality check classifier can be performed in real-time or in a cadence during the session and the features can be assembled and/or processed by logistic regression ML model circuits at the end of the session (or in a cadence during the session). It is understood that logistic regression is just one example of an ML model and other models can be used such as gradient-boosted trees, random forests, neural networks, support vector machines, etc.

As described herein, in some examples, the sleep/wake classification can be improved by providing additional details regarding the sleep state. For example, instead of a binary classification of intervals as awake or asleep, the classification can provide sub-categories of sleep. For example, the sleep can be classified as REM sleep, non-REM sleep stage one, non-REM sleep stage two, or non-REM sleep stage three. In some examples, one or more of the non-REM sleep stages can be combined (e.g., merged) to reduce the number of states and simply the display. In some such examples, the sleep states can include awake, REM sleep, or non-REM sleep. In some such examples, the sleep states can include awake, REM sleep, non-REM sleep stages one or two (e.g., combining sleep stage one and sleep stage two), or non-REM stage three. In some such examples, the sleep states can include awake, REM sleep, non-REM sleep stages two or three (e.g., combining sleep stage two and sleep stage three), or non-REM stage one. In some examples, as described herein, the sleep tracking results can be displayed or reported to the user. The additional detail regarding the sleep state can provide more robust information for sleep tracking and evaluating quality of sleep.

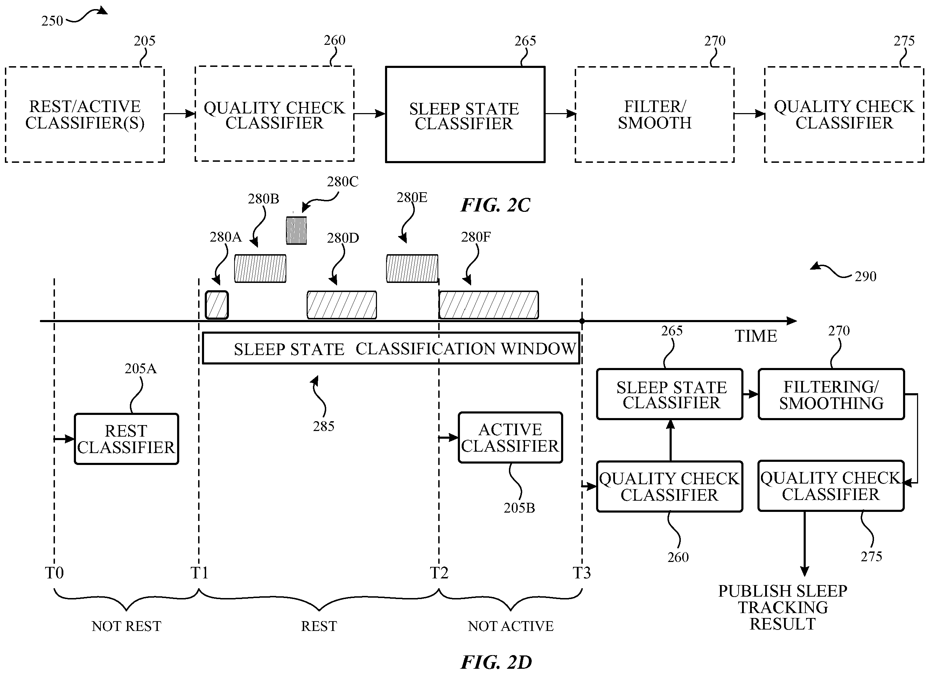

FIGs. 2C-2D illustrate an example block diagram and corresponding timing diagram for sleep tracking (e.g., sleep state classification) according to examples of the disclosure. FIG. 2C illustrates an example block diagram 250 of processing circuitry for sleep tracking of according to examples of the disclosure. The processing circuitry can include a digital signal processor (e.g., corresponding to DSP 109 in FIG. 1B) and/or one or more additional processors (e.g., corresponding to processor(s) 108). In some examples, the processing circuitry can include a programmable logic device (PLD), field programmable gate array (FPGA), or other logic device. The processing circuitry can include a rest/active classifier 205, a first quality check classifier 260, a sleep state classifier 265, a smoothing/filtering post-processor 270, and a second quality check classifier 275. The classifications and/or filtering/smoothing can be implemented in hardware, software, firmware, or any combination thereof.

Rest/active classifier 205 in block diagram 250 can be the same as or similar to the rest/active classifier 205 described with reference to block diagram 200, the details of which are omitted for brevity. Rest/active classifier 205 can be used to define a start time and an end time for a sleep tracking session.

First quality check classifier 260 can be optionally included for sleep tracking to estimate/classify the quality of the sensor data (e.g., using one or more features extracted during the sleep session for use in the sleep state classification). The quality of the sensor data can be indicative of the wearable device being on-wrist during the sleep tracking session, and can establish a confidence in the sleep state classification. In some examples, the quality check by first quality check classifier 260 can correspond to process 600, the details of which are not repeated for brevity. Additionally or alternatively, the quality check by first quality check classifier 260 can determine whether the sleep session lasted a for a threshold duration (e.g., 1 hour, 2 hours, 4 hours, etc.), as the confidence in the sleep state classifications improves for a sleep session longer than the threshold duration compared with a sleep session shorter than the threshold duration. In some examples, when the criteria are satisfied for the quality check of the first quality check classifier 260 (e.g., the device meets the on-wrist criterion and/or the sleep session meets the threshold duration criterion), the sleep classification by sleep state classifier 265 is performed. In some examples, when the criteria are not satisfied for the quality check of the first quality check classifier 260 (e.g., the device fails to meet the on-wrist criterion or the sleep session fails to meet the threshold duration criterion), the sleep classification by sleep state classifier 265 is not performed (e.g., thereby saving power). It is understood that, in some examples, when the classification by sleep state classifier 265 is not performed, that the results of the session are not displayed and/or stored. In some examples, a quality check for whether the device is on-wrist is performed only after the quality check determining that the sleep session duration meets or exceeds the threshold duration is satisfied.

Smoothing and filtering post-processor 270 can optionally be included to smooth/filter the sleep state classification. Smoothing and filtering post-processor 270 can be similar to smoothing and filtering post-processor 220, but with some differences to account for the difference in outputs of sleep state classifier 265 and sleep classifier 210. For example, smoothing and filtering post-processor 270 can also remove very short sleep intervals (e.g., to remove quiet wakefulness or other false-positive sleep intervals) as described with reference to FIGs. 7A-7B. However, smoothing and filtering post-processor 270 may additionally filter very short sleep intervals of a first sleep state (e.g., REM sleep) among immediately preceding and following sleep intervals of different sleep state(s) (e.g., non-REM sleep stage one, two or three). For example, similar to the description of first filter block 705, the output of the sleep state classifier can be filtered to remove very short sleep intervals of a particular sleep state (e.g., less than a threshold time such as 15 seconds, 30 seconds, 45 seconds, etc.) at any point in the session (e.g., across the entire classification window). These very short sleep state intervals may be false positives (high-frequency transients) and/or may represent sleep state intervals that are not sufficiently long for meaningful for understanding sleep/health benefits. These very short sleep state intervals may also be difficult to present to the user because these representations of less meaningful sleep information to user clutters the presentation of the more meaningful longer duration sleep state intervals in the sleep tracking result. Filtering out the very short sleep state intervals can include replacing the indication of a sleep state of the very short sleep intervals with an indication of the awake state or a different sleep state (e.g., depending on the state that precedes or follows a respective very short sleep interval). In some examples, the smoothing/filtering can be performed on the output of sleep state classifier 265 only after the second quality check by second quality check classifier 275 is satisfied (e.g., to avoid filtering/smoothing when the state classifications will not be displayed and/or stored).

FIG. 2D illustrates an example timing diagram 290 illustrating features and operation of the processing circuitry for sleep tracking according to examples of the disclosure. The timeline (e.g., times T1-T3), the operation of the rest classifier 205A and active classifier 205B (e.g., the rest/active classifier 205), criteria to start and termination of the sleep session, and classification window 235/285 described with respect to FIG. 2B are the same or similar to these corresponding elements in FIG. 2D, the details of which are not repeated for brevity.

The data in the sleep state classification window 285 can be processed by the sleep state classifier 265 as described in more detail with respect to process 800 and block diagram 500. In some examples, the sleep state classification by sleep state classifier 265 can begin in response to the end of the session (or a threshold period of time after the session or in response to a user request). In some examples, the sleep state classification by sleep state classifier 265 can begin only after the confidence in the session is satisfied as determined by the first quality check classifier 260 (e.g., saving power by avoiding processing when the first quality checks are not satisfied). In some examples, the sleep state classification by sleep state classifier 265 can begin (e.g., upon the end of the session), but can be aborted if ongoing, if the confidence in the session is not satisfied as determined by the first quality check classifier 260. In some examples, the sleep state classification estimating a user's sleep state can be stored in memory and/or displayed to the user. For example, sleep state classification estimating a user's sleep state can be displayed or stored as a sequence of sleep intervals (e.g., consecutive periods of time classified as a respective sleep state) represented by blocks 280A-280F as shown on the timeline in FIG. 2D. In some examples, the sleep states are presented on a display (e.g., touch screen 128). In some examples, the sleep states are presented on a timeline of different sleep states represented as sleep state intervals at different elevations. For example, blocks 280A, 280D and 280F can correspond to a first sleep state (e.g., non-REM sleep stage one), blocks 280B and 280E can correspond to a second sleep state (e.g., non-REM sleep stage two/three), and block 280C can correspond to a third sleep state (e.g., REM sleep). It is understood the awake state intervals may be represented by gaps in the timeline at which no other sleep state is represented. Alternatively, the awake state intervals may be represented by blocks at a different elevation. It is understood that although three elevations are shown in FIG. 3D, that more or fewer elevations and sleep states may be represented in the data displayed to the user (e.g., depending on how many sleep state outputs are output by sleep state classifier 265).

In some examples, the sleep state classification estimating a user's sleep states can be displayed and/or stored only when confidence in the session is satisfied as indicated by the first quality check classifier 260 and the second quality check classifier 275. In some examples, the sleep/wake classification estimating a user's sleep can be displayed and/or stored instead of the sleep state classification when confidence in the session as to sleep/wake classification is satisfied as indicated by the first quality check classifier 260 and the second quality check classifier 275 (when the quality check(s) do not establish confidence in the session as to the sleep state classification, but sufficient confidence in the session as to binary sleep/wake classification). In some examples, when confidence in the session is not satisfied as indicated by the first quality check classifier 260 and the second quality check classifier 275, the sleep state classification and/or sleep/wake state classification are not displayed and/or stored.

In some examples, the quality check by second quality check classifier 275 can include a determination if one or more criteria are satisfied by the classifications output from sleep state classifier 265. In some examples, the quality check by second quality check classifier 275 can determine whether the total sleep time for the sleep session lasted for a threshold duration (e.g., 1 hour, 2 hours, 3 hours, etc.), as the confidence in the sleep state classifications improves for a sleep session longer than the threshold duration compared with a sleep session shorter than the threshold duration.

In some examples, the threshold duration for second quality check classifier 275 can be shorter than the threshold duration for the first quality check classifier 260. Additionally or alternatively, the quality check by second quality check classifier 275 can determine whether the distribution of sleep states in the classification correspond to physiologically observed distributions of sleeps states (e.g., based on empirical measurement from sleep studies). In some such examples, the quality check can include determining whether the proportion (e.g., percentage) of total sleep time for the sleep session classified in a first sleep state (e.g., REM sleep) is less than a first threshold (e.g., 65%, 70%, etc.). In some such examples, the quality check can include determining whether the percentage of total sleep time for the sleep session classified in a second sleep state (e.g., non-REM sleep stage one) is less than a second threshold (e.g., 65%, 70%, etc.). The first threshold and second threshold can be determined from empirical measurement from sleep studies, for example. In some examples, the first and second thresholds can be the same. In some examples, the first and second thresholds can be the different. Although the above description evaluates two sleep states against a threshold (e.g., the first threshold and the second threshold), it is understood that, in some examples, fewer or more sleep states can be similarly evaluated against a threshold. In some examples, when the criteria are satisfied for the quality check of the second quality check classifier 275 (e.g., the total sleep time within the session meets the total sleep time criterion and/or the proportion of the total sleep time within one or more sleep states meets the corresponding threshold(s)), the sleep classification by sleep state classifier 265 can be stored and/or displayed. In some examples, when the criteria are not satisfied for the quality check of the second quality check classifier 275 (e.g., the total sleep time within the session fails to meet the total sleep time criterion or the proportion of the total sleep time within one or more sleep states fails to meet the corresponding threshold(s))), the sleep classification by sleep state classifier 265 is not stored and/or displayed, and optionally the sleep/wake binary classification is stored and/or displayed. When displaying the sleep/wake classification (binary classification), the data from sleep state classifier 265 can be merged (e.g., compressed) by merging the sleep intervals for all sleep states that are not the awake state into a single sleep state. In some examples, a quality check for whether the proportion of the total sleep time within one or more sleep states meets the corresponding threshold(s) is performed only after the quality check determining that the whether the total sleep time meets or exceeds the threshold duration is satisfied.

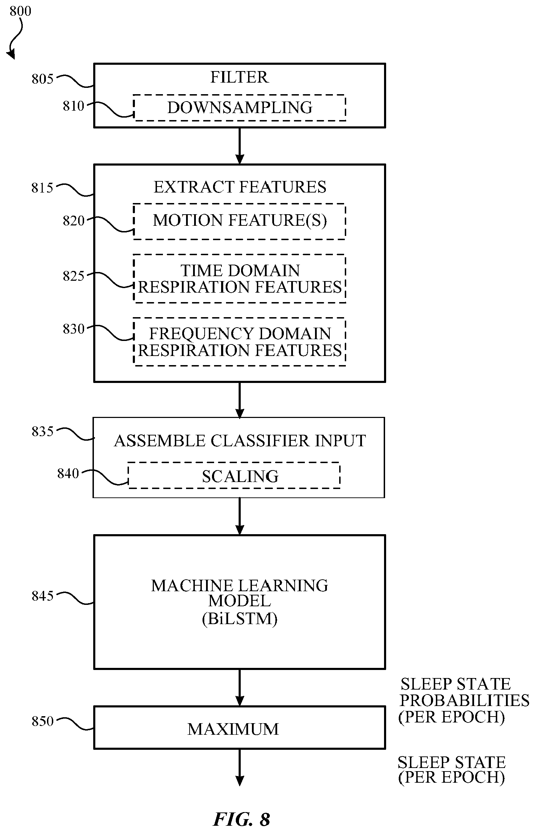

FIG. 8 illustrates an example process for a sleep state classifier according to examples of the disclosure. Process 800 can be performed by processing circuitry including processor(s) 108 and/or DSP 109. In some examples, process 800 can be performed partially in real-time (e.g., as sufficient data for processing is received), partially in a cadence during the session, and/or partially at the end of the session. In some examples, process 800 can be performed entirely at the end of the session (e.g., after the quality checks by first quality check classifier 260 are satisfied).

At 805, the sleep state classifier can optionally filter the data input into the classifier (e.g., sleep state classifier 265). In some examples, the motion data can also optionally be down-sampled at 810. At 815, the sleep state classifier can extract multiple features from the motion data, optionally including one or more motion features (820), one or more time-domain respiration features (825), and one or more frequency-domain respiration features (830). The multiple features can be computed for each epoch of motion data. Process 800 from 805-830 can be the same or similar to the description of process 400 from 405-430, the details of which are not repeated here for brevity. Additionally, the details of feature extraction, described in more detail herein with respect to FIG. 5, are not repeated here for brevity. However, it is understood that sleep/wake classification of process 400 and the sleep state classification of process 800 may rely on different set of extracted features. For example, the sleep state classification of process 800 may use some features that are not used for the sleep/wake classification of process 400 (or vice versa).

At 835, the input for the sleep state classifier can be assembled. The sleep state classifier input can be assembled from features for N epochs and can correspond to a longer duration period (e.g., corresponding to 5 minutes, 10 minutes, etc.). In some examples, the sleep state classifier input can be assembled from features for N epochs of the entire sleep session. In some examples, the input can include N*M features, where M features are extracted for each of N epochs. In some examples, the N epochs include an epoch of interest (e.g., the one for which the output classification applies) and N-1 epochs before and/or after the epoch of interest. In some examples, (N-1)/2 epochs before the epoch of interest are used as well as (N-1)/2 epochs after the epoch of interest. In some examples, the N-1 epochs may not be distributed evenly on both sides of the epoch of interest (e.g., 75% before and 25% after the epoch of interest). In some examples, the N-1 epochs before the epoch of interest are used. In some examples, the input can be compressed to reduce the number of features. For example, the features from multiple epochs can be reduced by sum-pooling the features for k consecutive epochs to reduce the input to $\frac{N}{k} * M$ features. A buffer can be used to store data (raw and/or filtered/down-sampled acceleration data and/or extracted features) corresponding to the longer duration period such that sufficient data can be available as input to the sleep state classifier.

In some examples, the features can also be scaled at 840. For example, the extracted features may have different ranges (e.g., maximum and minimum values) among other characteristics. In some examples, the scaling can transform the range for one or more of the features. In some examples, the scaling can transform the range for each of the features to be the same (e.g., a common range). In some examples, the scaling can include the use of a hyperbolic tangent function to map the range of values for a given feature to (-1:1). In some examples, the scaling can map the minimum and maximum values to the 1st and 95th percentile values and outliers outside the 95th percentile value can be outside the range of values (e.g., greater than 1 or less than -1). In some examples, the outliers may be treated with more care by the machine learning model or may decrease the confidence in the output of the machine learning model. It is understood that scaling to a range of values between -1 to 1 is a representative range, but that other ranges can be used (and optionally different ranges can be used for different features). Additionally, it is understood that the scaling may be achieved without using a hyperbolic tangent function. For example, scaling can be achieve using mean normalization or scaling to unit length, among other possibilities.

At 845, the classifier input can be processed with an ML model, such as a long-short term memory (LSTM) artificial neural network. In some examples, the LSTM neural network can be implemented as a bidirectional LSTM (BiLSTM) neural network (also referred to herein as a BiLSTM machine learning model). The bidirectional LSTM neural network can process the data from the end of the session to the start of the session and from the start of the session to the end of the session. In some examples, the BiLSTM neural networks includes one or more dense layers (also referred to as fully connected layers). In some examples, a first dense layer can be included to transform the classifier input before providing the one or more BiLSTM layers. In some examples, the first dense layer can increase the dimensionality of the input (e.g., the input dimensionality for features can be increased from M extracted features). In some examples, a second dense layer can be included to transform the output of the BiLSTM layers. In some examples, the second dense layer can reduce the dimensionality of the output (e.g., combining the information into a smaller dimensionality. Although a first dense layer is described before the BiLSTM layer(s) and a second dense layer is described after the BiLSTM layer(s), it is understood that multiple dense layers can be used to increase or decrease the dimensionality of the input to or output from the BiLSTM layer(s). In some examples, the second dense layer decreases the output of the BiLSTM layers to the same dimensionality as the assembled classifier input before the first dense layer. In some examples, a SoftMax layer is included to generate the output probabilities from the outputs of the BiLSTM layer(s) (e.g., after one or more dense layers). In some examples, a third dense layer after the second dense layer further decreases the dimensionality from the output of the second dense layer to improve the predictions by the SoftMax layer. It is understood that LSTM and BiLSTM neural networks are just examples of an ML model and other models can be used such as gradient-boosted trees, convolutional neural networks, random forests, logistical regressions, support vector machines, etc.

In some examples, the output of the ML model can be a confidence value representing the probability (between 0 and 1) that the user is in a specific sleep state. In some examples, the ML model can output a confidence value for each period of time corresponding to the duration of the epoch (e.g., using a sliding window on the data buffer) and for each supported sleep state (optionally excluding the awake state). For example, when the system supports five sleep states (e.g., awake, REM sleep, non-REM sleep stage one, non-REM sleep stage two, and non-REM sleep stage three), the output can include five probabilities for each epoch. As another example, when the system supports four sleep states (e.g., awake, REM sleep, non-REM sleep stage one, non-REM sleep stage two/three), the output can include four probabilities for each epoch. The sum of the probabilities for each sleep state within the epoch can sum to 1. The output of the ML model can be represented as an array of confidence values for each of the supported sleep states and for each epoch of data (optionally computed using a sliding window as described herein).

At 850, a maximum function can be applied to the output of the ML model to detect the highest probability sleep state for the epoch. For example, a wake state can be detected when the confidence value for the wake state is greatest, a REM sleep state can be detected when the confidence value for the REM sleep state is greatest, a non-REM sleep state stage one can be detected when the confidence value for the non-REM sleep state stage one is greatest, and so on. The output after maximizing can be an array of sleep state classifications (per epoch). The array of sleep state classifications can be displayed (optionally with some post-processing and in accordance with a quality check) as sleep state intervals (e.g., a sequence of sleep state and awake state periods) as described herein.

As discussed above, aspects in of the present technology include the gathering and use of physiological information. The technology may be implemented along with technologies that involve gathering personal data that relates to the user's health and/or uniquely identifies or can be used to contact or locate a specific person. Such personal data can include demographic data, date of birth, location-based data, telephone numbers, email addresses, home addresses, and data or records relating to a user's health or level of fitness (e.g., vital signs measurements, medication information, exercise information, etc.).

The present disclosure recognizes that a user's personal data, including physiological information, such as data generated and used by the present technology, can be used to the benefit of users. For example, assessing a user's sleep conditions (e.g., to determine a user's rest/active state and/or sleep/wake state) may allow a user to track or otherwise gain insights about their health.

The present disclosure contemplates that the entities responsible for the collection, analysis, disclosure, transfer, storage, or other use of such personal data will comply with well-established privacy policies and/or privacy practices.

In particular, such entities should implement and consistently use privacy policies and practices that are generally recognized as meeting or exceeding industry or governmental requirements for maintaining personal information data private and secure. Such policies should be easily accessible by users, and should be updated as the collection and/or use of data changes. Personal information from users should be collected for legitimate and reasonable uses of the entity and not shared or sold outside of those legitimate uses. Further, such collection/sharing should require receipt of the informed consent of the users. Additionally, such entities should consider taking any needed steps for safeguarding and securing access to such personal information data and ensuring that others with access to the personal information data adhere to their privacy policies and procedures. Further, such entities can subject themselves to evaluation by third parties to certify their adherence to widely accepted privacy policies and practices. The policies and practices may be adapted depending on the geographic region and/or the particular type and nature of personal data being collected and used.

Despite the foregoing, the present disclosure also contemplates embodiments in which users selectively block the collection of, use of, or access to, personal data, including physiological information. For example, a user may be able to disable hardware and/or software elements that collect physiological information. Further, the present disclosure contemplates that hardware and/or software elements can be provided to prevent or block access to personal data that has already been collected. Specifically, users can select to remove, disable, or restrict access to certain health-related applications collecting users' personal health or fitness data.

Therefore, according to the above, some examples of the disclosure are directed to a method. The method can comprise: extracting, for each of a plurality of epochs, a first plurality of features from first motion data from a multi-channel motion sensor and classifying, using the first plurality of features for the plurality of epochs, a state for each of the plurality of epochs as one of a plurality of sleep states (e.g., sleep state or awake state, or multiple sleep states). The first plurality of features can comprise one or more first motion features, one or more time-domain respiration features extracted from a first channel of a first stream of motion data derived from the first motion data, the first channel corresponding to a selected channel of the multi-channel motion sensor, and one or more frequency-domain respiration features extracted from a second channel of a second stream of motion data derived from the first motion data, the second channel corresponding to the selected channel of the multi-channel motion sensor. Additionally or alternatively to one or more of the examples disclosed above, in some examples, the multi-channel motion sensor can comprise a three-axis accelerometer. Additionally or alternatively to one or more of the examples disclosed above, in some examples, the method can further comprise: filtering the first motion data using a high-pass filter. The one or more first motion features can be extracted from the first motion data after filtering using the high-pass filter. Additionally or alternatively to one or more of the examples disclosed above, in some examples, the method can further comprise: filtering the first motion data using a band-pass filter to generate the first stream of motion data. Additionally or alternatively to one or more of the examples disclosed above, in some examples, the method can further comprise filtering the first motion data using a low-pass filter; and down-sampling the first motion data from a first sampling rate to a second sampling rate lower than the first sampling rate. Additionally or alternatively to one or more of the examples disclosed above, in some examples, the method can further comprise: for each epoch: converting the first motion data into a first frequency domain representation for a first channel of the multi-channel motion sensor, a second frequency domain representation for a second channel of the multi-channel motion sensor, and a third frequency domain representation for a third channel of the multi-channel motion sensor; and computing a first signal-to-noise ratio using the first frequency domain representation, a second signal-to-noise ratio using the second frequency domain representation, and a third signal-to-noise ratio using the third frequency domain representation. The selected channel can correspond to a respective channel of the first channel, the second channel, or the third channel with a maximum signal-to-noise ratio among the first signal-to-noise ratio, second signal-to-noise ratio and third signal-to-noise ratio. Additionally or alternatively to one or more of the examples disclosed above, in some examples, the method can further comprise: filtering the first motion data using a band-pass filter to generate the first stream of motion data; computing, for each of a plurality of windows of the first stream of motion data, a plurality of variances including a variance for each channel of the multi-channel motion sensor and a maximum variance among the plurality of variances; and in accordance with a determination that the maximum variance for a respective window of the plurality of windows exceeds a threshold, excluding samples corresponding to the respective window from the first channel of the first stream of motion data. Additionally or alternatively to one or more of the examples disclosed above, in some examples, the classifying can be performed by a logistic regression machine learning model. Additionally or alternatively to one or more of the examples disclosed above, in some examples, the method can further comprise: extracting, for each of a plurality of windows, a second plurality of features from second motion data from the multi-channel motion sensor; classifying the second plurality of features to estimate a plurality of resting state confidences, each of the plurality of resting state confidences corresponding to one of the plurality of windows; and in accordance with a determination that the plurality of resting state confidences satisfy one or more first criteria, measuring the first motion data from the multi-channel motion sensor. Additionally or alternatively to one or more of the examples disclosed above, in some examples, the one or more first criteria can include a criterion that is satisfied when a threshold number of the plurality of resting state confidences corresponding to consecutive windows exceed a confidence threshold. Additionally or alternatively to one or more of the examples disclosed above, in some examples, the method can further comprise: in accordance with satisfying one or more second criteria, extracting the second plurality of features from the second motion data; and in accordance with failing to satisfy the one or more second criteria, forgo extracting the second plurality of features from the second motion data. Additionally or alternatively to one or more of the examples disclosed above, in some examples, the one or more second criteria include: a first criterion that is satisfied a threshold period of time before a user-designated bedtime; a second criterion that is satisfied when a device including the multi-channel motion sensor is not charging; and/or a third criterion that is satisfied when the device including the multi-channel motion sensor is detected in contact with a body part. Additionally or alternatively to one or more of the examples disclosed above, in some examples, the method can further comprise: sum-pooling the second plurality of features for multiple of the plurality of windows. Additionally or alternatively to one or more of the examples disclosed above, in some examples, the method can further comprise: extracting, for each of a second plurality of windows, a third plurality of features from third motion data from the multi-channel motion sensor; classifying the third plurality of features to estimate a second plurality of resting state confidences, each of the second plurality of resting state confidences corresponding to one of the second plurality of windows; and in accordance with a determination that the second plurality of resting state confidences satisfy one or more second criteria, ceasing measuring the first motion data from the multi-channel motion sensor. Additionally or alternatively to one or more of the examples disclosed above, in some examples, the method can further comprise: classifying, using a subset of the first plurality of features, the first motion data as qualifying data or as non-qualifying data. The subset can comprise at least one of the one or more first motion features, at least one of the one or more time-domain respiration features, and at least one of the one or more frequency-domain respiration features. Additionally or alternatively to one or more of the examples disclosed above, in some examples, the method can further comprise: in accordance with classifying the first motion data as qualifying data, storing or displaying sleep intervals based on the classification of each of the plurality of epochs. Additionally or alternatively to one or more of the examples disclosed above, in some examples, the method can further comprise: identifying, using the classification of each of the plurality of epochs, one or more sleep intervals of consecutive epochs classified as a sleep state; and in accordance with a respective sleep interval of the one or more sleep intervals being shorter than a threshold number of consecutive epochs, reclassifying the consecutive epochs of the respective sleep interval from the sleep state to a wake state. Additionally or alternatively to one or more of the examples disclosed above, in some examples, the method can further comprise: estimating, using the first motion data, a transition from a first motion state to a second motion state. The second motion state can correspond to reduced motion relative to the first motion state. Additionally or alternatively to one or more of the examples disclosed above, in some examples, the method can further comprise: estimating the transition can comprise: computing a log scale of a motion feature of the one or more motion features extracted from the first motion data for each of the plurality of epochs; median-filtering the log scale of the one of motion feature for each of the plurality of epochs; and estimating the transition at an epoch at which a median-filtered, log scaled motion feature falls below a threshold. Additionally or alternatively to one or more of the examples disclosed above, in some examples, the method can further comprise: identifying, using the classification of each of the plurality of epochs, one or more sleep intervals of consecutive epochs classified as a sleep state; and in accordance with a respective sleep interval of the one or more sleep intervals prior to the estimated transition being shorter than a threshold number of consecutive epochs and having a sleep density less than a threshold, reclassifying the consecutive epochs of the respective sleep interval from the sleep state to a wake state.

Some examples of the disclosure are directed to a non-transitory computer readable storage medium. The non-transitory computer readable storage medium can store instructions, which when executed by an electronic device comprising processing circuitry, can cause the processing circuitry to perform any of the above methods. Some examples of the disclosure are directed to an electronic device comprising: processing circuitry; memory; and one or more programs. The one or more programs can be stored in the memory and configured to be executed by the processing circuitry. The one or more programs can include instructions for performing any of the above methods.

Some examples of the disclosure are directed to an electronic device. The electronic device can comprise: a motion sensor (e.g., a multi-channel motion sensor) and processing circuity coupled to the motion sensor. The processing circuitry can be programmed to: extract, for each of a plurality of epochs, a first plurality of features from first motion data from the multi-channel motion sensor, and classify, using the first plurality of features for the plurality of epochs, a state for each of the plurality of epochs as one of a plurality of sleep states. The first plurality of features can comprise: one or more first motion features; one or more time-domain respiration features extracted from a first channel of a first stream of motion data derived from the first motion data, the first channel corresponding to a selected channel of the multi-channel motion sensor; and one or more frequency-domain respiration features extracted from a second channel of a second stream of motion data derived from the first motion data, the second channel corresponding to the selected channel of the multi-channel motion sensor. Additionally or alternatively to one or more of the examples disclosed above, in some examples, the motion sensor comprises a three-axis accelerometer. Additionally or alternatively to one or more of the examples disclosed above, in some examples, the processing circuitry can be further programmed to: filter the first motion data using a high-pass filter. The one or more first motion features can be extracted from the first motion data after filtering using the high-pass filter. Additionally or alternatively to one or more of the examples disclosed above, in some examples, the processing circuitry can be further programmed to filter the first motion data using a band-pass filter to generate the first stream of motion data. Additionally or alternatively to one or more of the examples disclosed above, in some examples, the processing circuitry can be further programmed to: filter the first motion data using a low-pass filter; and down-sample the first motion data from a first sampling rate to a second sampling rate lower than the first sampling rate. Additionally or alternatively to one or more of the examples disclosed above, in some examples, the processing circuitry can be further programmed to: for each epoch: convert the first motion data into a first frequency domain representation for a first channel of the multi-channel motion sensor, a second frequency domain representation for a second channel of the multi-channel motion sensor, and a third frequency domain representation for a third channel of the multi-channel motion sensor; and compute a first signal-to-noise ratio using the first frequency domain representation, a second signal-to-noise ratio using the second frequency domain representation, and a third signal-to-noise ratio using the third frequency domain representation. The selected channel can correspond to a respective channel of the first channel, the second channel, or the third channel with a maximum signal-to-noise ratio among the first signal-to-noise ratio, second signal-to-noise ratio and third signal-to-noise ratio. Additionally or alternatively to one or more of the examples disclosed above, in some examples, the processing circuitry can be further programmed to filter the first motion data using a band-pass filter to generate the first stream of motion data; compute, for each of a plurality of windows of the first stream of motion data, a plurality of variances including a variance for each channel of the multi-channel motion sensor and a maximum variance among the plurality of variances; and in accordance with a determination that the maximum variance for a respective window of the plurality of windows exceeds a threshold, exclude samples corresponding to the respective window from the first channel of the first stream of motion data. Additionally or alternatively to one or more of the examples disclosed above, in some examples, the processing circuitry can include machine learning circuitry. The classifying can be performed by a logistic regression machine learning model. Additionally or alternatively to one or more of the examples disclosed above, in some examples, the processing circuitry can be further programmed to extract, for each of a plurality of windows, a second plurality of features from second motion data from the multi-channel motion sensor; classify the second plurality of features to estimate a plurality of resting state confidences, each of the plurality of resting state confidences corresponding to one of the plurality of windows; and in accordance with a determination that the plurality of resting state confidences satisfy one or more first criteria, measure the first motion data from the multi-channel motion sensor. Additionally or alternatively to one or more of the examples disclosed above, in some examples, the one or more first criteria can include a criterion that is satisfied when a threshold number of the plurality of resting state confidences corresponding to consecutive windows exceed a confidence threshold. Additionally or alternatively to one or more of the examples disclosed above, in some examples, the processing circuitry can be further programmed to: in accordance with satisfying one or more second criteria, extract the second plurality of features from the second motion data; and in accordance with failing to satisfy the one or more second criteria, forgo extracting the second plurality of features from the second motion data. Additionally or alternatively to one or more of the examples disclosed above, in some examples, the one or more second criteria can include: a first criterion that is satisfied a threshold period of time before a user-designated bedtime; a second criterion that is satisfied when a device including the multi-channel motion sensor is not charging; and/or a third criterion that is satisfied when the device including the multi-channel motion sensor is detected in contact with a body part. Additionally or alternatively to one or more of the examples disclosed above, in some examples, the processing circuitry can be further programmed to sum-pool the second plurality of features for multiple of the plurality of windows. Additionally or alternatively to one or more of the examples disclosed above, in some examples, the processing circuitry can be further programmed to: extract, for each of a second plurality of windows, a third plurality of features from third motion data from the multi-channel motion sensor; classify the third plurality of features to estimate a second plurality of resting state confidences, each of the second plurality of resting state confidences corresponding to one of the second plurality of windows; and in accordance with a determination that the second plurality of resting state confidences satisfy one or more second criteria, cease measuring the first motion data from the multi-channel motion sensor. Additionally or alternatively to one or more of the examples disclosed above, in some examples, the processing circuitry can be further programmed to classify, using a subset of the first plurality of features, the first motion data as qualifying data or as non-qualifying data. The subset can comprise at least one of the one or more first motion features, at least one of the one or more time-domain respiration features, and at least one of the one or more frequency-domain respiration features. Additionally or alternatively to one or more of the examples disclosed above, in some examples, the processing circuitry can be further programmed to in accordance with classifying the first motion data as qualifying data, store and/or display sleep intervals based on the classification of each of the plurality of epochs. Additionally or alternatively to one or more of the examples disclosed above, in some examples, the processing circuitry can be further programmed to: identify, using the classification of each of the plurality of epochs, one or more sleep intervals of consecutive epochs classified as a sleep state; and in accordance with a respective sleep interval of the one or more sleep intervals being shorter than a threshold number of consecutive epochs, reclassify the consecutive epochs of the respective sleep interval from the sleep state to a wake state. Additionally or alternatively to one or more of the examples disclosed above, in some examples, the processing circuitry can be further programmed to estimate, using the first motion data, a transition from a first motion state to a second motion state. The second motion state can correspond to reduced motion relative to the first motion state. Additionally or alternatively to one or more of the examples disclosed above, in some examples, estimating the transition can comprise: computing a log scale of a motion feature of the one or more motion features extracted from the first motion data for each of the plurality of epochs; median-filtering the log scale of the one of motion feature for each of the plurality of epochs; and estimating the transition at an epoch at which a median-filtered, log scaled motion feature falls below a threshold. Additionally or alternatively to one or more of the examples disclosed above, in some examples, the processing circuitry can be further programmed to: identify, using the classification of each of the plurality of epochs, one or more sleep intervals of consecutive epochs classified as a sleep state; and in accordance with a respective sleep interval of the one or more sleep intervals prior to the estimated transition being shorter than a threshold number of consecutive epochs and having a sleep density less than a threshold, reclassify the consecutive epochs of the respective sleep interval from the sleep state to a wake state.

Some examples of the disclosure are directed to an electronic device. The electronic device can comprise: a motion sensor (e.g., a multi-channel motion sensor) and processing circuity coupled to the motion sensor. The processing circuitry can be programmed to: extract, for each of a plurality of epochs in a session, a first plurality of features from first motion data from the multi-channel motion sensor, and in accordance with a determination that one or more first criteria are satisfied, classify, using the first plurality of features for the plurality of epochs, a state for each of the plurality of epochs as one of a plurality of sleep states. The first plurality of features can comprise: one or more first motion features; one or more time-domain respiration features extracted from a first channel of a first stream of motion data derived from the first motion data, the first channel corresponding to a selected channel of the multi-channel motion sensor; and one or more frequency-domain respiration features extracted from a second channel of a second stream of motion data derived from the first motion data, the second channel corresponding to the selected channel of the multi-channel motion sensor. The plurality of sleep states can include a first sleep state corresponding to a wake state, a second sleep state corresponding to a rapid eye movement sleep state, and a third sleep state corresponding to one or more non-rapid eye movement sleep states. Additionally or alternatively to one or more of the examples disclosed above, in some examples, the third sleep state can correspond to first-stage non-rapid eye movement sleep state. The plurality of sleep states can include a fourth sleep state corresponding to a second-stage non-rapid eye movement sleep state and a third-stage non-rapid eye movement sleep state. Additionally or alternatively to one or more of the examples disclosed above, in some examples, the third sleep state can correspond to first-stage non-rapid eye movement sleep state. The plurality of sleep states can include a fourth sleep state corresponding to a second-stage non-rapid eye movement sleep state, and the plurality of sleep states can include a fifth sleep state corresponding to a third-stage non-rapid eye movement sleep state. Additionally or alternatively to one or more of the examples disclosed above, in some examples, the processing circuitry can be further programmed to: in accordance with a determination that the one or more first criteria are not satisfied, forgo classifying the state for each of the plurality of epochs. Additionally or alternatively to one or more of the examples disclosed above, in some examples, the one or more first criteria can include a criterion that is satisfied when the session is longer than a threshold duration. Additionally or alternatively to one or more of the examples disclosed above, in some examples, the one or more first criteria can include a criterion that is satisfied when the electronic device including the multi-channel motion sensor is detected in contact with a body part during the session. Additionally or alternatively to one or more of the examples disclosed above, in some examples, detecting the electronic device including the multi-channel motion sensor is in contact with the body part during the session can be based on a subset of the first plurality of features including at least one of the one or more first motion features, at least one of the one or more time-domain respiration features, and at least one of the one or more frequency-domain respiration features. Additionally or alternatively to one or more of the examples disclosed above, in some examples, the processing circuitry can be further programmed to: in accordance with a determination that one or more second criteria are satisfied, store or display sleep intervals based on classification of each of the plurality of epochs. The sleep intervals can include a sleep interval corresponding to the first sleep state, a sleep interval corresponding to the second sleep state, and a sleep interval corresponding to the third sleep state. Additionally or alternatively to one or more of the examples disclosed above, in some examples, the one or more second criteria can include a criterion that is satisfied when a total duration of the epochs classified different than the first sleep state is greater than a threshold duration. Additionally or alternatively to one or more of the examples disclosed above, in some examples, the one or more second criteria can include a criterion that is satisfied when a proportion of a total duration of the epochs classified as corresponding to the second sleep state to the total duration of the epochs classified different than the first sleep state is less than a first threshold proportion. Additionally or alternatively to one or more of the examples disclosed above, in some examples, the one or more second criteria can include a criterion that is satisfied when a proportion of a total duration of the epochs classified as corresponding to the third sleep state to the total duration of the epochs classified different than the first sleep state is less than a second threshold proportion. Additionally or alternatively to one or more of the examples disclosed above, in some examples, the processing circuitry can be further programmed to: in accordance with a determination that one or more third criteria are satisfied, store or display sleep intervals based on the classification of each of the plurality of epochs. Sleep intervals corresponding to the second sleep state and sleep interval corresponding to the third sleep state can be merged. Additionally or alternatively to one or more of the examples disclosed above, in some examples, the one or more third criteria can include a criterion that is satisfied when: a total duration of the epochs classified different than the first sleep state is less than a threshold duration; a proportion of a total duration of the epochs classified as corresponding to the second sleep state to the total duration of the epochs classified different than the first sleep state is greater than a first threshold proportion; or a proportion of a total duration of the epochs classified as corresponding to the third sleep state to the total duration of the epochs classified different than the first sleep state is greater than a second threshold proportion. Additionally or alternatively to one or more of the examples disclosed above, in some examples, the processing circuitry can be further programmed to: in accordance with a determination that the one or more second criteria and the one or more third criteria are not satisfied, forgo storing or displaying the sleep intervals based on the classification of each of the plurality of epochs. Additionally or alternatively to one or more of the examples disclosed above, in some examples, the processing circuitry can include machine learning circuitry. Classifying can be performed by a bidirectional long-short-term-memory machine learning model. Additionally or alternatively to one or more of the examples disclosed above, in some examples, the processing circuitry can be further programmed to: scale the first plurality of features to a common range of values for use by the bidirectional long-short-term-memory machine learning model. Additionally or alternatively to one or more of the examples disclosed above, in some examples, the machine learning circuitry can be configured to output a probability for each of the plurality of sleep states for each of the plurality of epochs, and can configured to classify the state for each of the plurality of epochs using a maximum among the probability for each of the plurality of sleep states for each of the plurality of epochs. Additionally or alternatively to one or more of the examples disclosed above, in some examples, the processing circuitry can be further programmed to: identify, using classification of each of the plurality of epochs, a first sleep interval of consecutive epochs classified as a respective sleep state of the plurality of sleep states preceded by a second sleep interval of consecutive epochs classified as a different respective sleep state and followed by a third sleep interval of consecutive epochs classified as the different respective sleep state; and in accordance with the first sleep interval being shorter than a threshold number of consecutive epochs, reclassify the consecutive epochs of the first sleep interval from the respective sleep state to the different respective sleep state. Additionally or alternatively to one or more of the examples disclosed above, in some examples, the processing circuitry can be further programmed to: estimate, using the first motion data, a transition from a first motion state to a second motion state. The second motion state can correspond to reduced motion relative to the first motion state. Additionally or alternatively to one or more of the examples disclosed above, in some examples, estimating the transition can comprise: computing a log scale of a motion feature of the one or more motion features extracted from the first motion data for each of the plurality of epochs; median-filtering the log scale of the one of motion feature for each of the plurality of epochs; and estimating the transition at an epoch at which a median-filtered, log scaled motion feature falls below a threshold. Additionally or alternatively to one or more of the examples disclosed above, in some examples, the processing circuitry can be further programmed to: identify, using the classification of each of the plurality of epochs, one or more sleep intervals of consecutive epochs classified as the second sleep state or the third sleep state; and in accordance with a respective sleep interval of the one or more sleep intervals prior to the transition being shorter than a threshold number of consecutive epochs and having a sleep density less than a sleep density threshold, reclassify the consecutive epochs of the respective sleep interval from the second sleep state or the third sleep state to the first sleep state. Additionally or alternatively to one or more of the examples disclosed above, in some examples, the multi-channel motion sensor comprises a three-axis accelerometer. Additionally or alternatively to one or more of the examples disclosed above, in some examples, the processing circuitry can be further programmed to: filter the first motion data using a high-pass filter. The one or more first motion features can be extracted from the first motion data after filtering using the high-pass filter. Additionally or alternatively to one or more of the examples disclosed above, in some examples, the processing circuitry can be further programmed to: filter the first motion data using a band-pass filter to generate the first stream of motion data. Additionally or alternatively to one or more of the examples disclosed above, in some examples, the processing circuitry can be further programmed to: filter the first motion data using a low-pass filter; and down-sample the first motion data from a first sampling rate to a second sampling rate lower than the first sampling rate. Additionally or alternatively to one or more of the examples disclosed above, in some examples, the processing circuitry can be further programmed to: for each epoch: convert the first motion data into a first frequency domain representation for a first channel of the multi-channel motion sensor, a second frequency domain representation for a second channel of the multi-channel motion sensor, and a third frequency domain representation for a third channel of the multi-channel motion sensor. Additionally or alternatively to one or more of the examples disclosed above, in some examples, the processing circuitry can be further programmed to: for each epoch: compute a first signal-to-noise ratio using the first frequency domain representation, a second signal-to-noise ratio using the second frequency domain representation, and a third signal-to-noise ratio using the third frequency domain representation. The selected channel can correspond to a respective channel of the first channel, the second channel, or the third channel with a maximum signal-to-noise ratio among the first signal-to-noise ratio, second signal-to-noise ratio and third signal-to-noise ratio. Additionally or alternatively to one or more of the examples disclosed above, in some examples, the processing circuitry can be further programmed to: filter the first motion data using a band-pass filter to generate the first stream of motion data; compute, for each of a plurality of windows of the first stream of motion data, a plurality of variances including a variance for each channel of the multi-channel motion sensor and a maximum variance among the plurality of variances; and in accordance with a determination that the maximum variance for a respective window of the plurality of windows exceeds a threshold, exclude samples corresponding to the respective window from the first channel of the first stream of motion data.

Some examples of the disclosure are directed to a method. The method can comprise: extracting, for each of a plurality of epochs in a session, a first plurality of features from first motion data from a multi-channel motion sensor. The first plurality of features can comprise: one or more first motion features; one or more time-domain respiration features extracted from a first channel of a first stream of motion data derived from the first motion data, the first channel corresponding to a selected channel of the multi-channel motion sensor; and one or more frequency-domain respiration features extracted from a second channel of a second stream of motion data derived from the first motion data, the second channel corresponding to the selected channel of the multi-channel motion sensor. The method can comprise: in accordance with a determination that one or more first criteria are satisfied, classifying, using the first plurality of features for the plurality of epochs, a state for each of the plurality of epochs as one of a plurality of sleep states. The plurality of sleep states can include a first sleep state corresponding to a wake state, a second sleep state corresponding to a rapid eye movement sleep state, and a third sleep state corresponding to one or more non-rapid eye movement sleep states. Additionally or alternatively to one or more of the examples disclosed above, in some examples, the third sleep state can correspond to first-stage non-rapid eye movement sleep state. The plurality of sleep states can include a fourth sleep state corresponding to a second-stage non-rapid eye movement sleep state and a third-stage non-rapid eye movement sleep state. Additionally or alternatively to one or more of the examples disclosed above, in some examples, the third sleep state can correspond to first-stage non-rapid eye movement sleep state. The plurality of sleep states can include a fourth sleep state corresponding to a second-stage non-rapid eye movement sleep state, and the plurality of sleep states can include a fifth sleep state corresponding to a third-stage non-rapid eye movement sleep state. Additionally or alternatively to one or more of the examples disclosed above, in some examples, the method can further comprise: in accordance with a determination that the one or more first criteria are not satisfied, forgoing classifying the state for each of the plurality of epochs. Additionally or alternatively to one or more of the examples disclosed above, in some examples, the one or more first criteria can include a criterion that is satisfied when the session is longer than a threshold duration. Additionally or alternatively to one or more of the examples disclosed above, in some examples, the one or more first criteria can include a criterion that is satisfied when the electronic device including the multi-channel motion sensor is detected in contact with a body part during the session. Additionally or alternatively to one or more of the examples disclosed above, in some examples, detecting the electronic device including the multi-channel motion sensor is in contact with the body part during the session can be based on a subset of the first plurality of features including at least one of the one or more first motion features, at least one of the one or more time-domain respiration features, and at least one of the one or more frequency-domain respiration features. Additionally or alternatively to one or more of the examples disclosed above, in some examples, the method can further comprise: in accordance with a determination that one or more second criteria are satisfied, storing or displaying sleep intervals based on classification of each of the plurality of epochs. The sleep intervals can include a sleep interval corresponding to the first sleep state, a sleep interval corresponding to the second sleep state, and a sleep interval corresponding to the third sleep state. Additionally or alternatively to one or more of the examples disclosed above, in some examples, the one or more second criteria can include a criterion that is satisfied when a total duration of the epochs classified different than the first sleep state is greater than a threshold duration. Additionally or alternatively to one or more of the examples disclosed above, in some examples, the one or more second criteria can include a criterion that is satisfied when a proportion of a total duration of the epochs classified as corresponding to the second sleep state to the total duration of the epochs classified different than the first sleep state is less than a first threshold proportion. Additionally or alternatively to one or more of the examples disclosed above, in some examples, the one or more second criteria can include a criterion that is satisfied when a proportion of a total duration of the epochs classified as corresponding to the third sleep state to the total duration of the epochs classified different than the first sleep state is less than a second threshold proportion. Additionally or alternatively to one or more of the examples disclosed above, in some examples, the method can further comprise: in accordance with a determination that one or more third criteria are satisfied, storing or displaying sleep intervals based on the classification of each of the plurality of epochs. Sleep intervals corresponding to the second sleep state and sleep interval corresponding to the third sleep state can be merged. Additionally or alternatively to one or more of the examples disclosed above, in some examples, the one or more third criteria can include a criterion that is satisfied when: a total duration of the epochs classified different than the first sleep state is less than a threshold duration; a proportion of a total duration of the epochs classified as corresponding to the second sleep state to the total duration of the epochs classified different than the first sleep state is greater than a first threshold proportion; or a proportion of a total duration of the epochs classified as corresponding to the third sleep state to the total duration of the epochs classified different than the first sleep state is greater than a second threshold proportion. Additionally or alternatively to one or more of the examples disclosed above, in some examples, the method can further comprise: in accordance with a determination that the one or more second criteria and the one or more third criteria are not satisfied, forgoing storing or displaying the sleep intervals based on the classification of each of the plurality of epochs. Additionally or alternatively to one or more of the examples disclosed above, in some examples, classifying can be performed by a bidirectional long-short-term-memory machine learning model. Additionally or alternatively to one or more of the examples disclosed above, in some examples, the method can further comprise: scaling the first plurality of features to a common range of values for use by the bidirectional long-short-term-memory machine learning model. Additionally or alternatively to one or more of the examples disclosed above, in some examples, the method can further comprise: estimating a probability for each of the plurality of sleep states for each of the plurality of epochs, and classifying the state for each of the plurality of epochs using a maximum among the probability for each of the plurality of sleep states for each of the plurality of epochs. Additionally or alternatively to one or more of the examples disclosed above, in some examples, the method can further comprise: identifying, using classification of each of the plurality of epochs, a first sleep interval of consecutive epochs classified as a respective sleep state of the plurality of sleep states preceded by a second sleep interval of consecutive epochs classified as a different respective sleep state and followed by a third sleep interval of consecutive epochs classified as the different respective sleep state; and in accordance with the first sleep interval being shorter than a threshold number of consecutive epochs, reclassifying the consecutive epochs of the first sleep interval from the respective sleep state to the different respective sleep state. Additionally or alternatively to one or more of the examples disclosed above, in some examples, the method can further comprise: estimating, using the first motion data, a transition from a first motion state to a second motion state. The second motion state can correspond to reduced motion relative to the first motion state. Additionally or alternatively to one or more of the examples disclosed above, in some examples, estimating the transition can comprise: computing a log scale of a motion feature of the one or more motion features extracted from the first motion data for each of the plurality of epochs; median-filtering the log scale of the one of motion feature for each of the plurality of epochs; and estimating the transition at an epoch at which a median-filtered, log scaled motion feature falls below a threshold. Additionally or alternatively to one or more of the examples disclosed above, in some examples, the method can further comprise: identifying, using the classification of each of the plurality of epochs, one or more sleep intervals of consecutive epochs classified as the second sleep state or the third sleep state; and in accordance with a respective sleep interval of the one or more sleep intervals prior to the transition being shorter than a threshold number of consecutive epochs and having a sleep density less than a sleep density threshold, reclassifying the consecutive epochs of the respective sleep interval from the second sleep state or the third sleep state to the first sleep state. Additionally or alternatively to one or more of the examples disclosed above, in some examples, the multi-channel motion sensor comprises a three-axis accelerometer. Additionally or alternatively to one or more of the examples disclosed above, in some examples, the method can further comprise: filtering the first motion data using a high-pass filter. The one or more first motion features can be extracted from the first motion data after filtering using the high-pass filter. Additionally or alternatively to one or more of the examples disclosed above, in some examples, the method can further comprise: filtering the first motion data using a band-pass filter to generate the first stream of motion data. Additionally or alternatively to one or more of the examples disclosed above, in some examples, the method can further comprise: filtering the first motion data using a low-pass filter; and down-sampling the first motion data from a first sampling rate to a second sampling rate lower than the first sampling rate. Additionally or alternatively to one or more of the examples disclosed above, in some examples, the method can further comprise: for each epoch: converting the first motion data into a first frequency domain representation for a first channel of the multi-channel motion sensor, a second frequency domain representation for a second channel of the multi-channel motion sensor, and a third frequency domain representation for a third channel of the multi-channel motion sensor. Additionally or alternatively to one or more of the examples disclosed above, in some examples, the method can further comprise: for each epoch: computing a first signal-to-noise ratio using the first frequency domain representation, a second signal-to-noise ratio using the second frequency domain representation, and a third signal-to-noise ratio using the third frequency domain representation. The selected channel can correspond to a respective channel of the first channel, the second channel, or the third channel with a maximum signal-to-noise ratio among the first signal-to-noise ratio, second signal-to-noise ratio and third signal-to-noise ratio. Additionally or alternatively to one or more of the examples disclosed above, in some examples, the method can further comprise: filtering the first motion data using a band-pass filter to generate the first stream of motion data; computing, for each of a plurality of windows of the first stream of motion data, a plurality of variances including a variance for each channel of the multi-channel motion sensor and a maximum variance among the plurality of variances; and in accordance with a determination that the maximum variance for a respective window of the plurality of windows exceeds a threshold, excluding samples corresponding to the respective window from the first channel of the first stream of motion data.

Some examples of the disclosure are directed to a non-transitory computer readable storage medium. The non-transitory computer readable storage medium can store instructions, which when executed by an electronic device comprising processing circuitry, can cause the processing circuitry to perform any of the above methods. Some examples of the disclosure are directed to an electronic device comprising: processing circuitry; memory; and one or more programs.

The one or more programs can be stored in the memory and configured to be executed by the processing circuitry. The one or more programs can include instructions for performing any of the above methods.

Although examples of this disclosure have been fully described with reference to the accompanying drawings, it is to be noted that various changes and modifications will become apparent to those skilled in the art. Such changes and modifications are to be understood as being included within the scope of examples of this disclosure as defined by the appended claims.

**Claims**

1. A method comprising:

   extracting, for each of a plurality of epochs in a session, a first plurality of features from first motion data from a multi-channel motion sensor, wherein the first plurality of features comprises:

      one or more first motion features;
      one or more time-domain respiration features extracted from a first channel of a first stream of motion data derived from the first motion data, the first channel corresponding to a selected channel of the multi-channel motion sensor; and
      one or more frequency-domain respiration features extracted from a second channel of a second stream of motion data derived from the first motion data, the second channel corresponding to the selected channel of the multi-channel motion sensor; and

   in accordance with a determination that one or more first criteria are satisfied, classifying, using the first plurality of features for the plurality of epochs, a state for each of the plurality of epochs as one of a plurality of sleep states, the plurality of sleep states including a first sleep state corresponding to a wake state, a second sleep state corresponding to a rapid eye movement sleep state, and a third sleep state corresponding to one or more non-rapid eye movement sleep states.

2. The method of claim 1, wherein the third sleep state corresponds to first-stage non-rapid eye movement sleep state and wherein the plurality of sleep states includes a fourth sleep state corresponding to a second-stage non-rapid eye movement sleep state and a third-stage non-rapid eye movement sleep state.

3. The method of claim 1, wherein the third sleep state corresponds to a first-stage non-rapid eye movement sleep state, wherein the plurality of sleep states includes a fourth sleep state corresponding to a second-stage non-rapid eye movement sleep state, and wherein the plurality of sleep states includes a fifth sleep state corresponding to a third-stage non-rapid eye movement sleep state.

4. The method of claim 1, further comprising:
   in accordance with a determination that the one or more first criteria are not satisfied, forgoing classifying the state for each of the plurality of epochs.

5. The method of claim 4, wherein the one or more first criteria include a criterion that is satisfied when the session is longer than a threshold duration.

6. The method of claim 4, wherein the one or more first criteria include a criterion that is satisfied when an electronic device including the multi-channel motion sensor is detected in contact with a body part during the session.

7. The method of claim 1, further comprising:
   in accordance with a determination that one or more second criteria are satisfied, storing or displaying sleep intervals based on classification of each of the plurality of epochs, wherein the sleep intervals include a sleep interval corresponding to the first sleep state, a sleep interval corresponding to the second sleep state, and a sleep interval corresponding to the third sleep state.

8. The method of claim 7, wherein the one or more second criteria include a criterion that is satisfied when a total duration of the epochs classified different than the first sleep state is greater than a threshold duration.

9. The method of claim 7, further comprising:

in accordance with a determination that one or more third criteria are satisfied, storing or displaying sleep intervals based on the classification of each of the plurality of epochs, wherein sleep intervals corresponding to the second sleep state and sleep interval corresponding to the third sleep state are merged.

10. The method of claim 1, wherein classifying is performed by a bidirectional long-short-term-memory machine learning model.

11. The method of claim 10, further comprising:
scaling the first plurality of features to a common range of values for use by the bidirectional long-short-term-memory machine learning model.

12. The method of claim 10, further comprising:
estimating a probability for each of the plurality of sleep states for each of the plurality of epochs, and classifying the state for each of the plurality of epochs using a maximum among the probability for each of the plurality of sleep states for each of the plurality of epochs.

13. The method of claim 1, further comprising:

identifying, using classification of each of the plurality of epochs, a first sleep interval of consecutive epochs classified as a respective sleep state of the plurality of sleep states preceded by a second sleep interval of consecutive epochs classified as a different respective sleep state and followed by a third sleep interval of consecutive epochs classified as the different respective sleep state; and
in accordance with the first sleep interval being shorter than a threshold number of consecutive epochs, reclassifying the consecutive epochs of the first sleep interval from the respective sleep state to the different respective sleep state.

14. A non-transitory computer readable storage medium storing instructions, which when executed by an electronic device including processing circuitry, cause the processing circuitry to perform a method of any of claims 1-13.

15. An electronic device comprising:

a multi-channel motion sensor; and
processing circuity coupled to the multi-channel motion sensor, the processing circuitry programmed to perform a method of any of claims 1-13.

**FIG. 1A**

STRAP 146

WEARABLE DEVICE 100

TOUCH SCREEN 128

STRAP 146

100

**DEVICE**

106 ACCELEROMETER

110 COMMUNICATION CIRCUITY

124

118 PERIPHERAL DEVICE

105a A/D CONVERTER

108 PROCESSOR(S)

122 MICROPHONE/ SPEAKER

109 DIGITAL SIGNAL PROCESSOR

105b A/D CONVERTER

128 TOUCH SCREEN

102 LIGHT EMITTER

104 LIGHT SENSOR

111 PROGRAM STORAGE

114

**FIG. 1B**

FIG. 2A

FIG. 2B

250

| REST/ACTIVE CLASSIFIER(S) 205 | → | QUALITY CHECK CLASSIFIER 260 | → | SLEEP STATE CLASSIFIER 265 | → | FILTER/ SMOOTH 270 | → | QUALITY CHECK CLASSIFIER 275 |

*FIG. 2C*

290

SLEEP STATE CLASSIFICATION WINDOW

285

TIME

280A 280B 280C 280D 280E 280F

REST CLASSIFIER 205A

ACTIVE CLASSIFIER 205B

SLEEP STATE CLASSIFIER 265

FILTERING/ SMOOTHING 270

QUALITY CHECK CLASSIFIER 260

QUALITY CHECK CLASSIFIER 275

PUBLISH SLEEP TRACKING RESULT

T0        T1                 T2           T3

NOT REST          REST          NOT ACTIVE

*FIG. 2D*

300

305 — FILTER

310 — DOWNSAMPLING

315 — EXTRACT FEATURES

320 — MAGNITUDE
(PER SAMPLE),
VARIANCE
(PER WINDOW)

325 — ASSEMBLE
CLASSIFIER INPUT

330 — MACHINE LEARNING
MODEL

(LOGISTIC REGRESSION)

REST/ACTIVE
PROBABILITY          (PER WINDOW)

335 — THRESHOLD

REST/ACTIVE
STATE            (PER WINDOW)

*FIG. 3*

400

FILTER

405

DOWNSAMPLING

410

↓

EXTRACT FEATURES

415

MOTION FEATURE(S)

420

TIME DOMAIN
RESPIRATION FEATURES

425

FREQUENCY DOMAIN
RESPIRATION FEATURES

430

↓

ASSEMBLE CLASSIFIER INPUT

435

↓

MACHINE LEARNING
MODEL
(LOGISTIC REGRESSION)

440

↓ SLEEP PROBABILITY
(PER EPOCH)

THRESHOLD

445

↓ SLEEP/WAKE STATE
(PER EPOCH)

*FIG. 4*

**FIG. 5**

600

EXTRACTED FEATURES

605 — ASSEMBLE CLASSIFIER INPUT

610 — MACHINE LEARNING
MODEL
(LOGISTIC REGRESSION)

QUALITY
PROBABILITY

615 — THRESHOLD

QUALITY
CHECK RESULT

*FIG. 6*

FEATURE(S) ──→ ┌─────────────┐ 710
               │   IN-BED    │──── IN-BED TIME ────┐
               │  DETECTION  │                     │
               └─────────────┘                     │
                                                   ▼
                  705                              715
SLEEP/WAKE    ┌─────────────┐      ┌─────────────┐
CLASSIFICATION│FILTER SHORT │      │FILTER SHORT │
──────────────→│SLEEP INTERVALS│──→│SLEEP INTERVALS│──→ FILTERED SLEEP/
              │    FOR      │      │PRIOR TO BEING│    WAKE CLASSIFICATION
              │   SESSION   │      │   IN-BED    │
              └─────────────┘      └─────────────┘

*FIG. 7A*

*FIG. 7B*

EP 4 285 818 A1

800

| | FILTER |
|---|---|
| 805 | |
| 810 | DOWNSAMPLING |

| | EXTRACT FEATURES |
|---|---|
| 815 | |
| 820 | MOTION FEATURE(S) |
| 825 | TIME DOMAIN RESPIRATION FEATURES |
| 830 | FREQUENCY DOMAIN RESPIRATION FEATURES |

| | ASSEMBLE CLASSIFIER INPUT |
|---|---|
| 835 | |
| 840 | SCALING |

845 — MACHINE LEARNING MODEL (BiLSTM)

SLEEP STATE PROBABILITIES (PER EPOCH)

850 — MAXIMUM

SLEEP STATE (PER EPOCH)

*FIG. 8*

Europäisches
Patentamt

European
Patent Office

Office européen
des brevets

# EUROPEAN SEARCH REPORT

Application Number

EP 23 17 6151

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/337634 A1 (MCDARBY GARETH [IE] ET AL) 29 October 2020 (2020-10-29) | 1-5,7-9, 14,15 | INV. A61B5/113 |
| Y | * paragraphs [0053], [0059], [0067], | 6,10-12 | A61B5/00 |
| A | [0287]; figure 11 * | 13 | G16H50/00 |
| X | US 2021/038087 A1 (HENEGHAN CONOR [IE] ET AL) 11 February 2021 (2021-02-11) * paragraphs [0044], [0105], [0106] * | 1-5,7-9, 14,15 | |
| Y | CN 111 407 262 A (BEIJING HAISI RUIGE TECH CO LTD) 14 July 2020 (2020-07-14) | 10-12 | |
| A | * abstract * | 1,14,15 | |
| Y | EP 3 875 026 A1 (KONINKLIJKE PHILIPS NV [NL]) 8 September 2021 (2021-09-08) | 6 | |
| A | * paragraph [0018]; figure 1 * | 1-5,7-15 | |
| A | US 2020/009349 A1 (SHOULDICE REDMOND [IE] ET AL) 9 January 2020 (2020-01-09) * paragraph [0206] * | 1-15 | |

TECHNICAL FIELDS
SEARCHED      (IPC)

A61B
G16H

The present search report has been drawn up for all claims

| Place of search | Date of completion of the search | Examiner |
|---|---|---|
| Berlin | 13 October 2023 | Clevorn, Jens |

## ANNEX TO THE EUROPEAN SEARCH REPORT
## ON EUROPEAN PATENT APPLICATION NO.

EP 23 17 6151

This annex lists the patent family members relating to the patent documents cited in the above-mentioned European search report.
The members are as contained in the European Patent Office EDP file on
The European Patent Office is in no way liable for these particulars which are merely given for the purpose of information.

13-10-2023

| Patent document cited in search report | | Publication date | Patent family member(s) | | Publication date |
|---|---|---|---|---|---|
| US 2020337634 | A1 | 29-10-2020 | AU | 2013318046 A1 | 12-03-2015 |
| | | | AU | 2016204561 A1 | 21-07-2016 |
| | | | CA | 2883656 A1 | 27-03-2014 |
| | | | CN | 104812300 A | 29-07-2015 |
| | | | CN | 107647868 A | 02-02-2018 |
| | | | EP | 2897526 A1 | 29-07-2015 |
| | | | EP | 3912553 A1 | 24-11-2021 |
| | | | JP | 6404819 B2 | 17-10-2018 |
| | | | JP | 6742377 B2 | 19-08-2020 |
| | | | JP | 7078676 B2 | 31-05-2022 |
| | | | JP | 2015532855 A | 16-11-2015 |
| | | | JP | 2019048054 A | 28-03-2019 |
| | | | JP | 2020171797 A | 22-10-2020 |
| | | | KR | 20150058344 A | 28-05-2015 |
| | | | KR | 20200032248 A | 25-03-2020 |
| | | | NZ | 630602 A | 28-10-2016 |
| | | | NZ | 725344 A | 27-04-2018 |
| | | | US | 2015230750 A1 | 20-08-2015 |
| | | | US | 2020337634 A1 | 29-10-2020 |
| | | | WO | 2014047310 A1 | 27-03-2014 |
| US 2021038087 | A1 | 11-02-2021 | AU | 2007256872 A1 | 13-12-2007 |
| | | | CA | 2654095 A1 | 13-12-2007 |
| | | | CN | 101489478 A | 22-07-2009 |
| | | | EP | 2020919 A2 | 11-02-2009 |
| | | | EP | 3616611 A1 | 04-03-2020 |
| | | | HK | 1135868 A1 | 18-06-2010 |
| | | | JP | 2009538720 A | 12-11-2009 |
| | | | US | 2009203972 A1 | 13-08-2009 |
| | | | US | 2014163343 A1 | 12-06-2014 |
| | | | US | 2021038087 A1 | 11-02-2021 |
| | | | US | 2023293021 A1 | 21-09-2023 |
| | | | WO | 2007143535 A2 | 13-12-2007 |
| CN 111407262 | A | 14-07-2020 | NONE | | |
| EP 3875026 | A1 | 08-09-2021 | CN | 115209801 A | 18-10-2022 |
| | | | EP | 3875026 A1 | 08-09-2021 |
| | | | EP | 4114264 A1 | 11-01-2023 |
| | | | US | 2021275090 A1 | 09-09-2021 |
| | | | WO | 2021175630 A1 | 10-09-2021 |
| US 2020009349 | A1 | 09-01-2020 | AU | 2014287372 A1 | 21-01-2016 |
| | | | CN | 105592777 A | 18-05-2016 |
| | | | CN | 111467644 A | 31-07-2020 |
| | | | CN | 116328142 A | 27-06-2023 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 23 17 6151

This annex lists the patent family members relating to the patent documents cited in the above-mentioned European search report.
The members are as contained in the European Patent Office EDP file on
The European Patent Office is in no way liable for these particulars which are merely given for the purpose of information.

13-10-2023

| Patent document cited in search report | Publication date | Patent family member(s) | | Publication date |
|---|---|---|---|---|
| | | EP | 3019073 A2 | 18-05-2016 |
| | | EP | 4133997 A1 | 15-02-2023 |
| | | JP | 6698521 B2 | 27-05-2020 |
| | | JP | 7238194 B2 | 13-03-2023 |
| | | JP | 2016532481 A | 20-10-2016 |
| | | JP | 2022119930 A | 17-08-2022 |
| | | JP | 2023080065 A | 08-06-2023 |
| | | US | 2016151603 A1 | 02-06-2016 |
| | | US | 2020009349 A1 | 09-01-2020 |
| | | US | 2023248935 A1 | 10-08-2023 |
| | | WO | 2015006364 A2 | 15-01-2015 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63365840
- US 30938623